(19) Europäisches Patentamt
European Patent Office
Office européen des brevets

(11) EP 3 318 547 A1

(12) EUROPEAN PATENT APPLICATION

(43) Date of publication:
**09.05.2018 Bulletin 2018/19**

(21) Application number: **17186879.7**

(22) Date of filing: **18.08.2017**

(51) Int Cl.:
***C07C 17/00*** (2006.01) ***C07C 17/14*** (2006.01)
***C07C 19/01*** (2006.01) ***C07C 19/07*** (2006.01)
***C07C 22/02*** (2006.01) ***C07C 22/04*** (2006.01)
***C07C 23/08*** (2006.01) ***C07C 23/10*** (2006.01)
***C07C 23/16*** (2006.01) ***C07C 23/28*** (2006.01)
***C07C 255/50*** (2006.01) ***C07C 69/78*** (2006.01)
***C07C 205/06*** (2006.01) ***C07C 67/307*** (2006.01)
***C07C 201/12*** (2006.01) ***C07B 39/00*** (2006.01)
***C07C 253/30*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **08.11.2016 EP 16197769**

(71) Applicant: **Justus-Liebig-Universität Gießen**
**35390 Gießen (DE)**

(72) Inventors:
- **Schreiner, Peter R.**
**35435 Wettenberg (DE)**
- **Combe, Sascha H.**
**35080 Bad Endbach (DE)**

(74) Representative: **Stumpf, Peter**
**c/o TransMIT GmbH**
**Kerkrader Strasse 3**
**35394 Gießen (DE)**

(54) **PROCESS FOR THE CHLORINATION AND IODINATION OF COMPOUNDS USING N-HYDROXYPHTHALIMIDE**

(57) The invention provides a process for the production of halogenated hydrocarbons comprising the usage of at least one halogenating agent which contains at least one halogen-atom per formular unit in combination with substituted or unsubstituted *N*-hydroxyphthalimide and a metal-containing salt-like compound or a carboxylic or sulfonic acid with or without solvent.

EP 3 318 547 A1

## Description

### Field of the invention

The invention is about a chlorination process for chemical compounds. The chlorination preferably occurs at benzylic or aliphatic positions in hydrocarbon molecules.

### Background of the technology

Benzylic and alkyl chlorides are used for the production of a very large variety and quantity of chemicals. Well known methods for producing benzylic and alkyl chlorides are the thermal side-chain chlorination using elemental chlorine, as well as the usage of numerous other chlorinating agents, e.g., $SO_2Cl_2$, NaOCl, $^{t}$BuOCl, $Et_4NH_4Cl$, benzyltrimethylammonium tetrachloroiodate (BTME), 1,3-dichloro-5,5-dimethylhydantoin and NaCl/HCl, all of them allowing the chlorination of aliphatic C-H bonds. These radical-type chlorination reactions require heating or irradiation, and some chlorination agents are explosive, toxic as well as highly corrosive. The once-common solvent for side-chain chlorination reactions, $CCl_4$, is now forbidden in many countries due to its carcinogenic nature. Chlorination using elemental chlorine often results in nearly inseparable multiple side-chain chlorinated products. To avoid polychlorination nearly all of the methods mentioned above need a very large excess of substrate or, in many instances, the substrate is used as solvent, which represents a major drawback. For that reason the substrates typically are restricted to liquids. An additional drawback especially when using NaOCl or $^{t}$BuOCl is the formation of oxygenated products.

Methods for the iodination of unactivated alkanes are scarce within the state of the art because the initiation and propagation steps with elemental iodine are highly endothermic and are not counterbalanced by the heat released from iodoalkane formation, the latter one being owed to the relatively low C-I bond dissociation energies. Furthermore the use of $I_2$ affords hydrogen iodide that may immediately reduce the iodoalkane to the corresponding alkane. Photochemically initiated io-dinations suffer from the cleavage of the C($sp^3$)-I bond due to n- to $\sigma^*$-excitation at about 250 nm and are therefore generally unfavorable. Therefore iodoalkanes are relatively expensive yet highly valuable reactants in organic synthesis.

Thus, controlled catalytic and safe halogenation reactions (for example chlorination reactions, bromination reactions and iodination reactions) are not known in the state of the art.

### Content of the invention

It is therefore the aim of the present invention to address the aforementioned issues. This task is solved by the characterising technical features of claim 1; the dependent claims reveal additional preferred embodiments of the invention.

The invention revealed herein provides a safe, cheap, atom economic, air and moisture tolerating approach for the side-chain halogenation (e.g. chlorination, bromination, iodination) of a range of arenes and alkanes with yields of up to 85% at 25-30 °C with halogenating agents like 1,3-diiodo-5,5-dimethylhydantoin (DIH), 3-iodo-1,5,5-trimethylhydantoin (3-ITMH), 1,3-dibromo-5,5-dimethylhydantoin and trichloroisocyanuric acid (TCCA) and *N*-hydroxyphthalimide **1** (NHPI) as radical initiator.

NHPI serves as a precursor for the phthalimido-*N*-oxyl **2** (PINO) radical (Scheme 1), which abstracts hydrogen atoms from C-H bonds and which is widely used in the oxidation of hydrocarbons, C-C as well as C-N couplings. PINO can be generated in the presence of some metal catalyst or a strong oxidant.

Scheme 1: Formation of PINO **2**

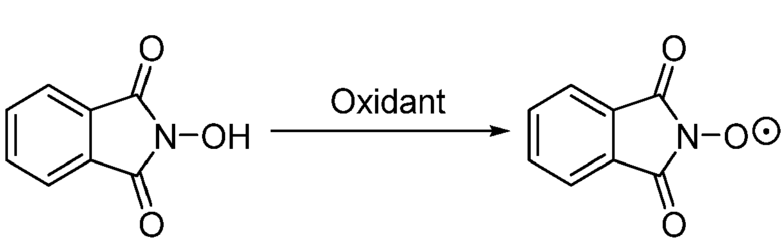


**1** **2**

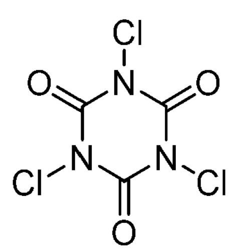

**TCCA**

TCCA is produced at a scale of about $10^6$ tons per year; it is mainly used as a disinfectant and in food chemistry.

All chlorine atoms in TCCA are typically utilized and it generally has a higher solubility than *N*-chlorosuccinimide (NCS), allowing highly concentrated reaction mixtures and reduction of the volume of organic solvent.

Table 1 (Fig. 1) shows the reaction scheme and the reaction conditions for the side-chain chlorination of toluene. Using $CH_2Cl_2$ as solvent affords 24% of benzyl chloride **4a** after 16 h (Table 1, entry 1). Adding two equivalents of $CH_3OH$ to the reaction or using AcOH as solvent increases the amount of core substitution product (Table 1, entry 2). It is found that 0.4 equiv. of TCCA is enough for the reaction to proceed smoothly. Adding *tert*-butyl hydroperoxide (TBHP) does not accelerate the reaction (Table 1, entry 3). In MeCN mainly **4b** and **4c** are produced (Table 1, entry 4). Increasing the solvent polarity increases the ability of the system for aromatic substitution (Table 1, entry 4), however, the use of DMF or DMSO as solvent leads to a violent reaction without formation of products, probably due to reagent decomposition. In $CHCl_3$ and 1,2-dichloroethane the reaction ends at about 45% (Table 1, entries 5 and 6), even after adding more TCCA. Equally unfortunate, the best results are obtained in $CCl_4$, giving mainly benzyl chloride **4a** and some benzal chloride **4d** (Table 1, entry 7). Variation of the amount of $Co(OAc)_2$ (Table 1, entries 7-10) shows that 2 mol% give the best results (Table 1, entry 7). Higher concentrations of $Co(OAc)_2$ have an adverse effect on the outcome of the reaction leading to lower conversion even after long reaction times (Table 1, entry 10). The cobalt catalyst is crucial for this reaction and no product forms in the absence of $Co(OAc)_2$ (Table 1, entry 11). The use of NCS only leads to quantitative recovery of the starting materials, even at higher dilution (Table 1, entries 12-13). This may be due to its lower solubility or lower oxidation potential as compared to TCCA.

The assumption that catalytically active trihalomethyl radicals (here $\bullet CCl_3$) are involved in the reaction is supported by performing the reaction in $CH_2Cl_2$ in the presence of $CBr_4$. Benzyl radicals can react with $CBr_4$ to form $\bullet CBr_3$ radicals and the corresponding bromide. The formation of benzyl bromide **5** confirms the mechanistic suggestion (Table 2, entry 1, cf. scheme 2). Surprisingly it is observed that $CBr_4$ catalyzes the first stage of the reaction (Table 2, entries 1-2). Testing catalytic amounts of $CBr_4$ (Table 2, entries 3-7) shows that with 10 mol% $CBr_4$ the reaction almost completes after 16 h (Table 2, entry 5). Lower and higher $CBr_4$ loadings (Table 2, entries 3-4 and 6-7) as well as using $HCBr_3$ instead of $CBr_4$ (Table 2, entries 8-11) or $HCCl_3$ (Table 2, entry 12) give inferior results. The reaction does not proceed with less than 5 mol% of NHPI (Table 2, entries 13-14). An NHPI loading of 10 mol% (Table 2, entry 5) is sufficient and gives nearly the same result as with higher catalyst loadings (Table 2, entry 15). Even higher TCCA loadings drastically slow down the reaction most likely due to decreased solubility (Table 2, entries 16-17), which is in agreement with the results of Table 1 (entry 1). Remarkably, the reaction still proceeds in the presence of water albeit somewhat more slowly (Table 2, entries 18-19). The reaction is temperature dependent and is best performed at ambient temperature (25 °C) (Table 2, entries 20-21). Finally, it is also possible to substitute $Co(OAc)_2$ for less toxic $Cu(OAc)_2$ (Table 2, entry 22).

Scheme 2: General reaction conditions for the side-chain chlorination of toluene (cf. Table 2)

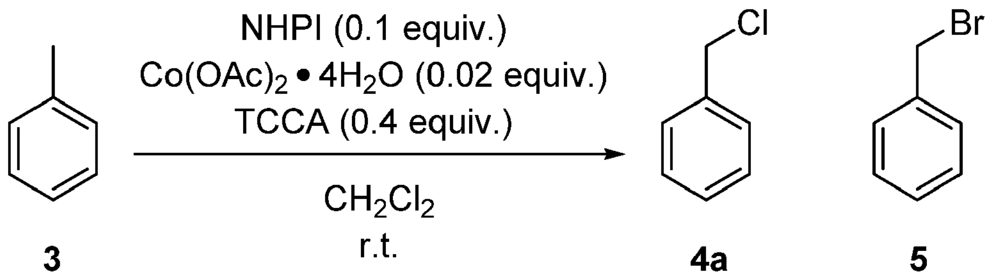


3 4a 5

With the optimized reaction conditions ($CBr_4$ (0.125 mmol), $CH_2Cl_2$ (2 mL), $Cu(OAC)_2\bullet H_2O$ (0.025 mmol), NHPI (0.125 mmol), substrate (1.25 mmol), TCCA (0.5 mmol), 25 °C, cf. general method P1), the scope of the reaction is shown as follows (cf. Fig. 3). It is well known to the person skilled in the art, that the performed experiments as shown in the following paragraphs do not confine the scope of the invention to these examples.

In general, side-chain chlorination provides good yields for arenes carrying an electron-withdrawing (50-85%) and a weakly electron-donating group (31-73%); modest to strong electron donating groups favor the $S_EAr$ reaction. The stabilization of the resulting substituted tolyl radicals plays an important role concerning the yield, giving the following experimentally observed order: p > *o* > m, which is consistent with benzyl radical stabilization. Deactivating groups destabilize the radical, hence the substitution at the meta-position gives the lowest yield. Electron-withdrawing groups adjacent to the radical also have a destabilizing effect mostly in the proximity to the radical center. Consequently, highest yields are obtained for the para-products. Unlike p-tolunitrile, however, no reaction occurrs with *o*- and m-tolunitrile even when $Co(OAc)_2$ is used instead of $Cu(OAc)_2$. In the case of methyl 3-(chloromethyl)benzoate **15** (product of applying the inventive halogenating process on methyl 3-(methyl)benzoate) the first direct side-chain chlorination is achieved.

Structure **15** is an important building block in the synthesis of taprostene, a stable prostacyclin analogue used as vasodilator. The chlorination of ethylbenzene gives a 2°/1° selectivity of 10:1. Cumene reacts sluggishly and gives an inseparable reaction mixture of core and side-chain chlorinated products. Adamantane reacts very fast to 1-chloroadamantane **18a** and 2-chloroadamantane **18b** in a 5:1 ratio (GC-MS), which is comparable to Cl-radical mediated adamantane chlorinations. Surprisingly, no oxygenated products are obtained for any substrate examined (nmr-studies show signal intensities related to oxygenated products only below 1%). The formation of doubly chlorinated products can be diminished to traces or even avoided by reducing the reaction time and by using two equivalents of the substrate. Doubling the amount of the substrate also increases the product yield as exemplified with p-tolunitrile.

Also, the C-H bond chlorination of cyclic and acyclic alkanes on gram scale is investigated (Scheme 3). As bromocyclohexane is obtained with $CBr_4$ as catalyst the latter is omitted. A reason for this observation may lie - without confining to a certain theory - in the differences of the bond dissociation energies of the products. The bond dissociation energy (BDE) difference for the $RCH_2$-Br bond in benzyl bromide (BDE = 63 kcal $mol^{-1}$) and bromoethane (BDE = 72 kcal $mol^{-1}$) is about 9 kcal $mol^{-1}$. This energy difference seems to allow homolytic cleavage of benzyl bromide (Table 2, entry 1). 2-Bromopropane has a BDE of about 74 kcal $mol^{-1}$, i.e., bromocyclohexane is stable under the reaction conditions. Cyclic substrates with various ring sizes (Fig. 3) are chlorinated in moderate yields (24-38%) and can be purified by simple distillation. The chlorination of n-hexane gives a mixture of three regioisomers, preferentially 3-chlorohexane **19a** and 2-chlorohexane **19b** (3:1).

Not being confined to a certain theory, the mechanism of the inventive chlorination process may be described - according to the well known state of the art and the revelation contained herein (e.g., ab-initio-computations at the M06-2X/cc-pVTZ and B3LYP-D3/6-31 G(d,p) level of theory) -as follows (cf. Fig. 5).

$Cu(OAc)_2$ and TCCA **23** generate the PINO radical **2** that abstracts an H• radical from **3** to form in an endergonic step ($\Delta G_{298}$ = 11.9 kcal $mol^{-1}$) the corresponding benzyl radical **24** (Initiation). The chain length of NHPI is *one* (Fig. 6A), which means that NHPI serves only as radical initiator. Radical **24** subsequently reacts with **23** to form product **4a** as well as **25** ($\Delta G_{298}$ = -15.8 kcal $mol^{-1}$). Radical **25** is also able to abstract an H• radical from **3** ($\Delta G_{298}$ =-37.4 kcal $mol^{-1}$) and forms **26;** the latter reacts further until all chlorine is consumed and cyanuric acid (**27**) precipitates from the reaction mixture as a white solid (catalytic cycle **B**). The computed reaction enthalpies for the hydrogen abstraction from **3** with dichloroisocy-anuric acid radical **25,** monochlorisocyanuric acid radical, cyanuric acid radical ($\Delta G_{298}$ = -26.1 to -27.6 kcal $mol^{-1}$) and $\bullet CBr_3$ radical ($\Delta G_{298}$ = -4.4 kcal $mol^{-1}$) show an overall highly exergonic process. The formation and precipitation of **27** is suggested to be the driving force of the reaction. $CBr_4$ catalyzes the reaction considerably (Fig. 2, entry 5) due to the favored formation of $\bullet CBr_3$ radicals ($\Delta G_{298}$ = - 9.3 kcal $mol^{-1}$). When all PINO is consumed trihalomethyl radicals carry on the chain reaction (Fig. 6B). Even if one equivalent of **24** is used to produce $\bullet CBr_3$ radicals, homolytic cleavage of **5** (Fig. 2, entry 1) as well as C-H abstraction of **3** by a $\bullet CBr_3$ radical gives overall two equivalents of **24** (catalytic cycle **A**).

The wide scope of the inventive process is also shown by the investigations which were made regarding the iodination (cf. Figs. 7 through 9 and schemes 3 through 6).

Using *N*-iodosuccinimide (NIS) in the reaction in place of TCCA gives benzyl iodide (Table 5, entry 1). DIH affords about twice as much product as compared to NIS (Table 5, entry 2). DIH is typically used in $S_EAr$ reactions; a reaction of toluene in combination with benzoyl peroxide does not give any side-chain iodinated product at all. Therefore DIH serves as a source for electrophilic "$I^+$". Remarkably, with DIH no metal catalyst is required and additionally a higher yield compared to NIS is obtained (Table 5, entry 6). DIH is a strong oxidant which is able to form PINO from NHPI. High dilution provides higher yields (Table 5, entries 3, 4, and 6). A NHPI loading of about 16 mol% is optimal for a smooth reaction (Table 5, entry 6). Lower NHPI loadings down to 8 mol% are lowering the yields (Table 5, entry 5) while higher loadings provide no additional benefit (Table 5 entries, 7 and 8). High substrate loadings also give higher yield (Table 5, entries 9 and 10). To activate the N-I bonds of DIH several carboxylic- and sulphonic acids are screened (Table 5, entries 12-23). In general, carboxylic acids afford much better yields than sulfonic acids, which can be correlated to their $pK_a$ values. Strong acids such as methane sulfonic acid lead to $S_EAr$ reactions.

Scheme 3: General reaction conditions for the side-chain iodination of toluene

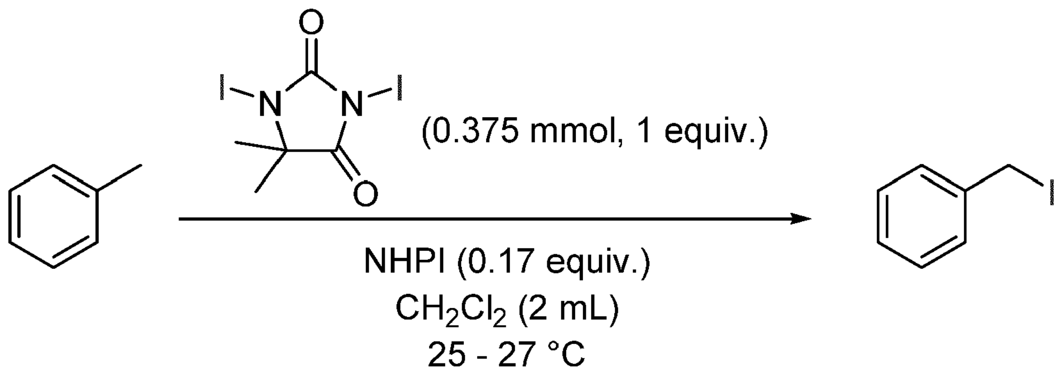


Scheme 4: Reaction to methyl 4-(iodomethyl)benzoate

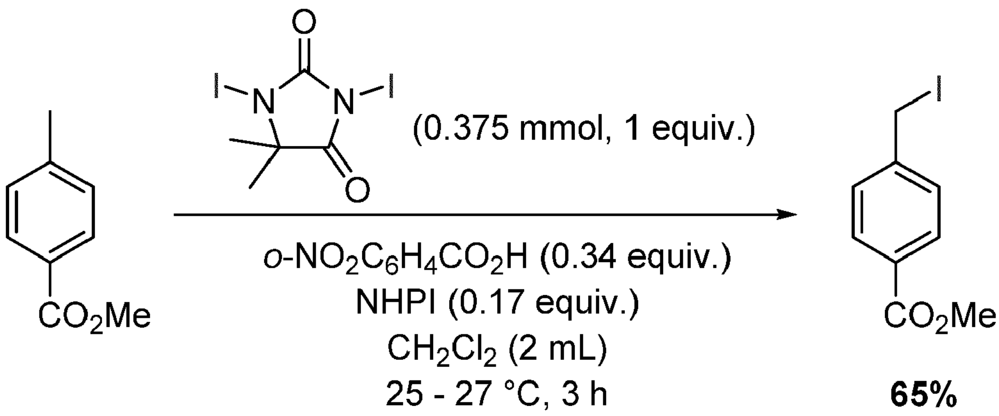


Fig. 8 shows some additional examples of iodine compounds accessible by use of the method revealed herein using NIS or DIH as iodine source. The examples of scheme 4/Fig. 8 and additional ones elaborated are shown in more detail in schemes 5a/5b and Fig. 9. Here, by use of the best reaction conditions so far elaborated (cf. Fig. 7, entry 18) the substrate scope of the reaction is shown by use of some examples, whereat the scope of the invention is not confined to the examples revealed herein.

Scheme 5a: General reaction conditions for the side-chain iodination of toluene with different iodination agents

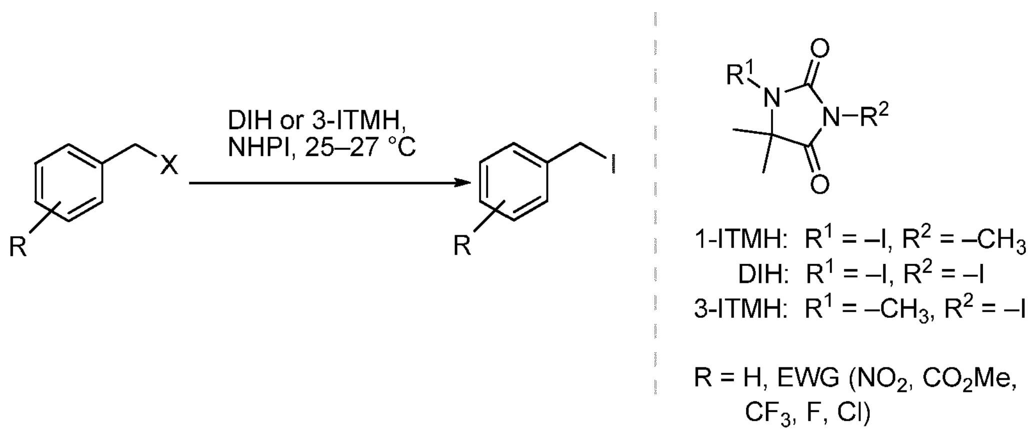

Scheme 5b: Reaction scheme of the investigation of the substrate scope for the side-chain iodination of toluene derivatives

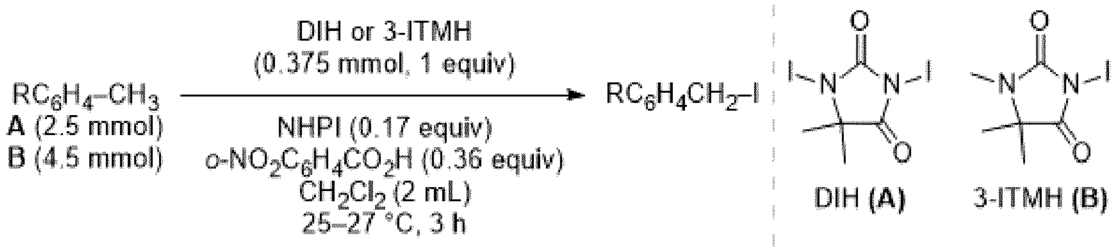


Arenes bearing an electron withdrawing or a weakly electron donating group give modest to good yields of isolated products (50-72%). This is the first process for the direct side-chain iodination of electron deficient arenes with isolation of the pure product. By virtue of the fact that electron-rich as well as electron-deficient arenes undergo an $S_EAr$ reaction in the presence of acid, this process is highly practical. Arenes bearing modest to strong electron donating groups form the corresponding benzylic nitroester; cyclohexene undergoes an addition reaction with $I_2$ followed by a ring opening to the corresponding ester. For cyclohexane an NMR-determined yield of about 15% is seen, while adamantane polymerizes. Ethylbenzene forms the corresponding benzylic nitroester in 24% isolated yield; p-bromoethylbenzene gives traces of the desired product and *p*-cyanoethylbenzene delivers 69% of the corresponding benzyl iodide **38**. The underlying reactivity is not merely a question of BDEs ($ArCH_2$-H: p-methyl anisole BDE = 87 kcal $mol^{-1}$; toluene= 88 kcal $mol^{-1}$) or radical stability (**34** vs. **38**), but rather a question of frontier orbital interaction. The results show that primarily arenes carrying an electron withdrawing group undergo a smooth reaction (low-lying SOMO). No $S_EAr$ products are detected for EWG containing arenes in preparative mass-balanced reactions; products and starting material (substrate) can be recovered with a mass loss of 7-10%.

Without being confined to a certain theory the mechanism for the iodination may be described as follows. The intermediate electrophilic radicals have to react with a nucleophilic iodine intermediate. The *geminal* dimethyl group may play an important role herein concerning its ability to push electron density towards the carbonyl group. The computational studies for the side-chain chlorination with TCCA show that the reaction proceeds preferentially via a carbonyl radical. The carbonyl radical abstracts a H-radical from the substrate forming an iminol, which tautomerizes to the amide. Another possibility may be the formation of a carbon centered radical, which is formed by decomposition of DIH. Matrix isolation of 1-ITMH reveals the formation of carbon centered radicals by decomposition. Small nucleophilic radicals can act as catalysts for the homolytic C-H bond cleavage of an electrophilic substrate; this phenomenon is defined as polarity reversal catalysis. Yields higher than 69% with DIH are not achieved so far, which may be an indication that not all iodine atoms in DIH are active and transferable. To support the assumption a NMR study with compounds 1-ITMH, DIH, and 3-ITMH is performed (Scheme 6). 1-ITMH gives the lowest yield (45%), while DIH and 3-ITMH behave similarly to each other (71% and 81% yield respectively). These results imply that the iodine next to the *geminal* dimethyl-group in DIH is mostly inactive. Therefore all the reactions are additionally performed with method **B** (Scheme 5b) substituting DIH with 3-ITMH. Increasing the substrate load, has the highest influence on the yields, as is evident from the data analysis revealed together with the examples below. In general, 3-ITMH affords similar results giving yields in the range of 50-72%. However 3-ITMH gives higher yields compared to DIH especially for substrates bearing a very strong deactivating group (**28, 34, 39**). Based on the results presented here and the known state of the art, but not being confined to a certain theory, the mechanism of the iodination may be as is shown in Fig. 10. With the use of DIH or 3-ITMH no metal catalyst is needed to start the reaction. Hence 3-ITMH must be able to oxidize NHPI (**1**) to PINO (**2**). PINO serves in the radical side-chain chlorination only as chain initiator and higher NHPI loadings do not show significant effects; an additional portion of NHPI after 1 h has no beneficial effect, thereby supporting the suggested mechanism. Also GC-MS analysis reveals the formation of phthalimide. The addition of acid increases the reaction rate considerably (Fig. 7, entry 18). The carboxylic acid serves as a proton source and also forms a halogen bond (complex **40**, cf. Fig. 11A-C) - both interactions polarize and weaken the N-I bond.

Scheme 6: Comparison of the iodine activity in **1**, **2** and **3** ($^1H$ NMR yield; dimethyl-terephthalate (0.625 M, 0.006 mmol) used as internal standard.)

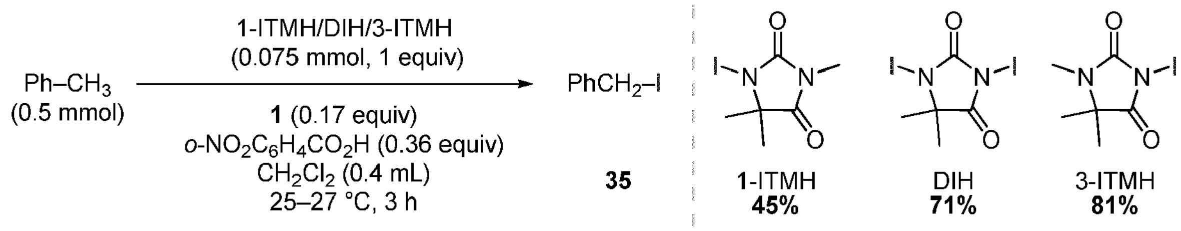


This dual binding mode is only seen when o-nitrobenzoic acid is used (Fig. 11A, **II** vs. **III**). The *N*-$CH_3$ group of 3-ITMH is shifted downfield more strongly as compared to the *geminal* dimethyl group (Fig. 11A, **III**). This may be due to steric hindrance or the fact that the carbamide group allows for better charge delocalization as compared to the amide group. A DFT study of DIH shows that protonation at the carbamide CO is about 6 kcal $mol^{-1}$ more stable than at the amide CO. In line with this complexation, the aromatic protons are shifted upfield, gaining electron density from 3-ITMH (Fig. 11A, **III** vs. **IV**). Carbon-13 NMR clearly shows that the iodine atom donates electron density into the carbonyl group of the acid (Fig. 11A, **III** vs. **IV**) resulting in a strong upfield shift. Complex formation is also supported by $^1H$ DOSY NMR (Fig. 11D and 11E). Both molecular components of the mixture are associated with the same diffusion coefficient value (which is lower than the values obtained for the free components in separate experiments). From Fig. 11E it is observed that the self-diffusion coefficients for the free molecular components are, at the least, half their values in the complex. It is observed that the acid suffers the greatest diffusion change when complexed (a factor of 2), a fact that agrees with its participation in the complex. The measurements are carried out in dilute solution in $CD_2Cl_2$, where viscosity variations due to small sample composition changes are negligible, thus the significant decrease in diffusion for o-nitrobenzoic acid when 3-ITMH is present, is a strong evidence for the existence of the complex in solution.

Furthermore, $I_2$ acts as a Lewis acid catalyst (halogen bond donor) but is not able to oxidize NHPI to PINO (Fig. 12A/B). 3-ITMH consumes *one* benzyl radical (**24**) and forms benzyl iodide (**35**) as well as a new radical **41**, which is able to carry on the radical chain (cycle **A**). The latter reacts further to trimethylhydantoin (**42**) forming a strong amide bond, which is the driving force of the reaction. This is strongly supported by the finding that upon synthesizing methyl 4-(iodomethyl)benzoate **30** compound **42** is isolated in 63% yield as by-product.

Generally no $S_EAr$ reactions are obtained for arenes bearing an electron-withdrawing group (EWG) under standard reaction conditions of the halogenation process revealed herein. The method enables convenient product isolation and gives moderate to good isolated yields (50-72%).

For further investigation of the scope of the halogenation process some substituted *N*-hydroxyphthalimides were tested as radical initiators (cf. scheme 7 and Figs. 13A,13B).

Scheme 7: General reaction conditions for the side-chain chlorination of toluene using different NHPI-derivatives

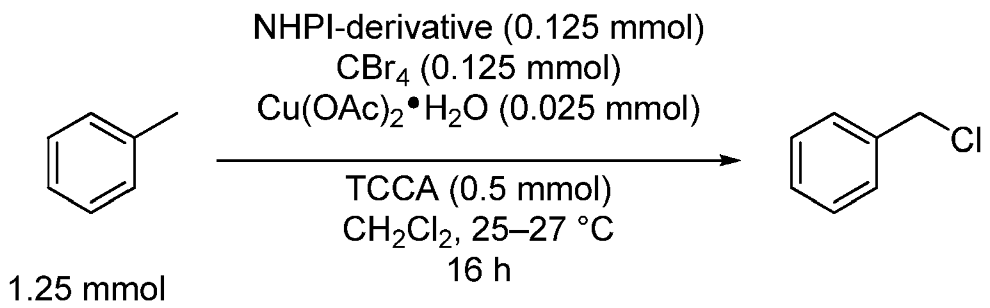


Schemes 8A and 8B show further examples with different halogenating agents of the new process of halogenation, additionally supporting the wide scope of the invention. Furthermore Scheme 8A clearly shows that TCCA is far more active compared to other chlorine sources (e.g. NIS and 1,3-dichloro-5,5-dimethylhydantoin).

Scheme 8A: Chlorination with 1,3-dichloro-5,5-dimethylhydantoin; $^1$H NMR yield based on toluene with DMT as internal standard

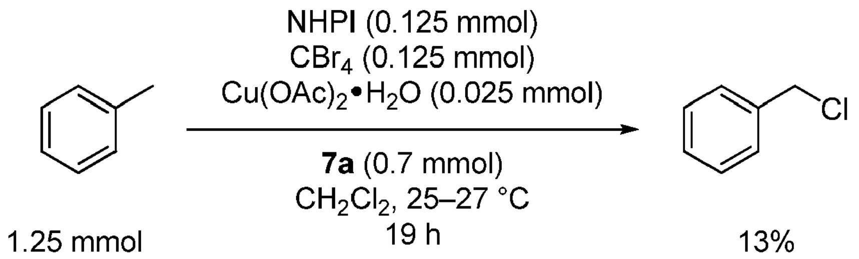


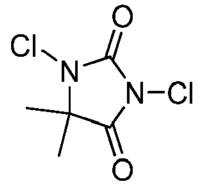

**7a**

Scheme 8B: Bromination with 1,3-dibromo-5,5-dimethylhydantoin; $^1$H NMR yield based on substrate with DMT as internal standard

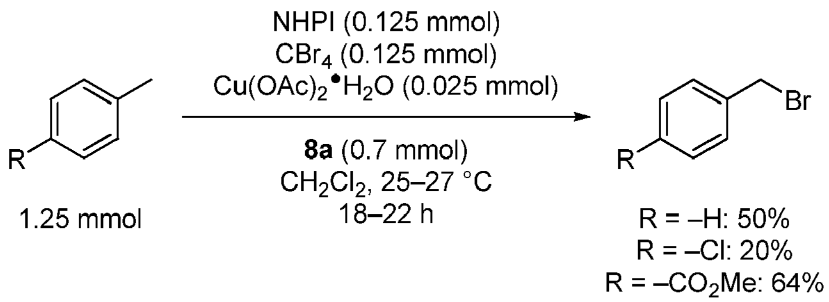


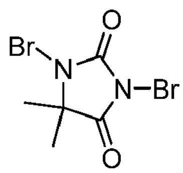

**8a**

In addition to exemplarily testing different halogenating agents a series of experients has been performed with a design of experiments (DoE) approach in order to show the optimization potential beyond the sample-experiments revealed herein so far (cf. schemes 9A/B and Figs. 14A/B and Figs. 15A/B/C).

Scheme 9A: First optimization of the yield with a design of experiments (DoE) approach

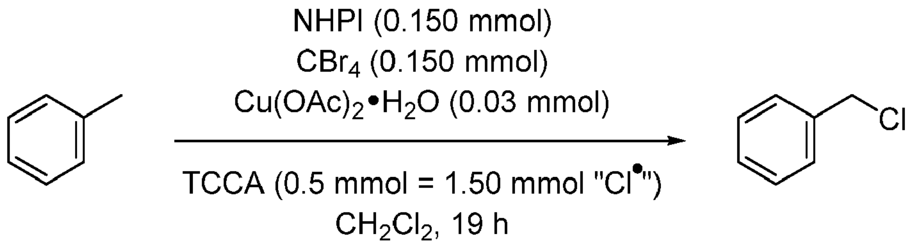

Scheme 9B: Second optimization of the yield with a design of experiments (DoE) approach

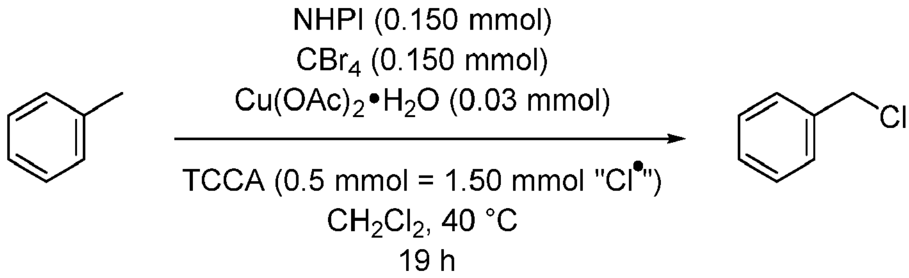


Thus, the invention provides a mild, safe, cheap, scalable, and controlled monohalogenation or mono pseudo-halogenation with only one equivalent of substrate and can be summarized as follows.

The invention is a process for the production of halogenated or pseudo-halogenated hydrocarbons using at least one halogenating or pseudo-halogenating agent which contains at least one halogen-atom or pseudo-halogen substituent per formular unit. It is generally characterized by the fact that at least one educt is reacted with at least one halogenating or pseudo-halogenating agent together with a substituted or unsubstituted *N*-hydroxyphthalimide and either a metal-containing salt-like compound or, alternatively, a carboxylic or sulfonic acid. The reaction mixture may additionally contain one or more solvents.

The halogenating agents according to the invention comprise at least one halogen atom per formular unit of either fluorine (F), chlorine (Cl), bromine (Br) or iodine (I).

The at least one halogenating agent according to the invention is chosen from the list comprising *N*-chlorosuccinimide, substituted *N*-chlorosuccinimide, trichloroisocyanuric acid, 1,3-dichloro-hydantoin, substituted 1,3-dichloro-hydantoin, 1-chloro-hydantoin, substituted 1-chloro-hydantoin, 3-chloro-hydantoin, substituted 3-chloro-hydantoin, *N*-bromosuccinimide, substituted *N*-bromosuccinimide, tribro-moisocyanuric acid, 1,3-dibromo-hydantoin, substituted 1,3-dibromo-hydantoin, 1-bromo-hydantoin, substituted 1-bromo-hydantoin, 3-bromo-hydantoin, substituted 3-bromo-hydantoin, *N*-iodosuccinimide, substituted *N*-iodosuccinimide, triiodoiso-cyanuric acid, 1,3-diiodo-hydantoin, substituted 1,3-diiodo-hydantoin, 1-iodo-hydantoin, substituted 1-iodo-hydantoin, 3-iodo-hydantoin, substituted 3-iodo-hydantoin. It is well known to the person skilled in the art that by substituting the halogenating agent the reactivity of the halogenating agent can be adapted to the educt in order to optimize the product selectivity of the process. Thus the at least one substituent of the substituted *N*-chlorosuccinimide or substituted *N*-bromosuccinimide or substituted *N*-iodosuccinimide or substituted 1,3-dichloro-hydantoin or substituted 1-chloro-hydantoin or substituted 3-chloro-hydantoin or substituted 1,3-dibromo-hydantoin or substituted 1-bromo-hydantoin or substituted 3-bromo-hydantoin or substituted 1,3-diiodo-hydantoin or substituted 1-iodo-hydantoin or substituted 3-iodo-hydantoin can be chosen from the list comprising the substituents methyl, ethyl, propyl, isopropyl, n-butyl, *sec*-butyl, *tert*-butyl, phenyl or any other suitable substituent.

Especially the following substituted halogenating agents can be used within the scope of the invention: 1,3-dichloro-5,5-dimethylhydantoin, 1-chloro-3,5,5-trimethylhydantoin, 1-chloro-3,3,4-trimethylhydantoin, 1-chloro-5,5-dimethylhydantoin, 1-chloro-3,3-dimethylhydantoin, 1,3-dibromo-5,5-dimethylhydantoin, 1-bromo-3,5,5-trimethylhydantoin, 1-bromo-3,3,4-trimethylhydantoin, 1-bromo-5,5-dimethylhydantoin, 1-bromo-3,3-dimethylhydantoin, 1,3-diiodo-5,5-dimethylhydantoin, 1-iodo-3,5,5-trimethylhydantoin, 1-iodo-3,3,4-trimethylhydantoin, 1-iodo-5,5-dimethylhydantoin, 1-iodo-3,3-dimethylhydantoin. This list is, of course, in no way understood to be limiting the scope of the invention.

It is well known to the person skilled in the art that there are several chemical substituents existing which are chemically behaving similar to halogen atoms. For example, "CN" is forming a dimer, called "Dicyane" (NC-CN) analogously to $F_2$, $Cl_2$, $Br_2$ and $I_2$. Also, often the "-CN" substituent can be introduced into organic molecules analogously to the introduction of halogen atoms, for example via $S_N2$-Substitution of alcohols using sodium cyanide. Therefore substituents which are in some ways behave similar to halogens are called "pseudo-halogens". Thus it is obvious to the person skilled in the art that the invention, developed by use of halogenating agents, is also comprising the usage of a pseudo-halogenating agent. Examples of the at least one pseudo-halogen substituent per formular unit of the pseudo-halogenating agent are RS-, -CN, -SCN, -NCS, -OCN, -NCO, -CNO, $-N_3$, - SeCN, whereat this list is not limiting the invention to the mentioned pseudo-halogenic substituents and R is chosen from the list comprising $-C_6H_5$, $-CH_3$.

The at least one pseudo-halogenating agent which can be used according to the invention is chosen from the list comprising *N*-(phenylthio)phthalimide, substituted *N*-(phenylthio)phthalimide, *N*-(methylthio)phthalimide, substituted *N*-(methylthio)phthalimide, *N*-thiocyanate-succinimide, substituted *N*-thiocyanate-succinimide, whereat the at least one substituent of the substituted *N*-(phenylthio)phthalimide or substituted *N*-(methylthio)phthalimide or substituted *N*-thiocyanate-succinimide is chosen from the list comprising the substituents methyl, ethyl, propyl, isopropyl, n-butyl, sec-

butyl, tert-butyl, phenyl, -OH, -OMe, -OEt, $-NH_2$, $-NR_aR_b$, $-NO_2$, $-CO_2R_c$ whereat $R_a$, $R_b$ and $R_c$ are independently from each other chosen from the List comprising methyl, ethyl, propyl, i-propyl, butyl, sec-butyl, tert-butyl.

The invention further comprises a Process according to one of the previously described examples or a combination of at least two of the previously described examples, characterized in that the metal-containing salt-like compound contains at least one metal-atom chosen from the metals of the groups 6, 7, 8, 9, 10, 11 or 12 of the periodic system of chemical elements. As examples of metal-containing salt-like compounds according to the invention cobalt(II)acetate, $Co(OAc)_2$, or copper(II)acetate, $Cu(OAc)_2$ can be used.

The invention further comprises a process where the carboxylic or sulfonic acid as revealed above exhibits a $pK_a$ value between -3 and 6, more preferred between -2 and 5, and most preferred between 3.5 and 1.2. Thus the carboxylic or sulfonic acid is preferably chosen from the list comprising the acids benzoic acid, acetic acid, diphenic acid, p-nitrobenzoic acid, o-nitrobenzoic acid, Camphor sulfonic acid, trifluoroacetic acid, p-toluenesulfonic acid. It is obvious to the person skilled in the art that this list is not understood to be confining the scope of the invention and that derivatives of carboxylic or sulfonic acids may also be applied, e.g., Acetic acid anhydride, halogenated acetic acid, e.g., diiodoacetic acid, dichloroacetic acid, trichloroacetic acid etc.

The substituted *N*-hydroxyphthalimide according to the invention contains at least one substituent chosen from the list comprising the substituents methyl, ethyl, propyl, isopropyl, n-butyl, *sec*-butyl, *tert*-butyl, phenyl, F, Cl, Br, I, $CF_3$, $NO_2$, CN, $CO_2Me$, OAc, $NHR_d$, NHAc, OH, OMe. $R_d$ is chosen from the List comprising methyl, ethyl, propyl, *i*-propyl, butyl, *sec*-butyl, *tert*-butyl. It is obvious to the person skilled in the art that if the substituted *N*-hydroxyphthalimide contains two or more substituents, they are chosen independently from each other from the above mentioned list.

It is also possible to apply the substrate/educt in excess; the more substrate/educt is applied, the better the yield. Therefore the amount of substituted or unsubstituted *N*-hydroxyphthalimide within the reaction mixture may be set either with respect to educt or to halogenating agent/pseudo-haogenating agent. Thus the amount of substituted or unsubstituted *N*-hydroxyphthalimide lies between 0.1 mol% and 50 mol%, more preferred between 1 mol% and 40 mol%, even more preferred between 2 mol% and 30 mol% and most preferred between 3 mol% and 20 mol% in relation to the educt or the halogenating or pseudo-halogenating agent.

In the same way the reaction mixture may also contain a certain amount of $CBr_4$ between 0.1 mol% and 50 mol%, more preferred between 1 mol% and 30 mol%, even more preferred between 5 mol% and 20 mol% and most preferred between 7 mol% and 15 mol% in relation to the educt or the halogenating or pseudo-halogenating agent.

It is obvious to the person skilled in the art that the concentration of the metal containing salt-like compound resp. the carboxylic or sulfonic acid in the reaction mixture can vary to a great extent, depending on the individual activity of the compound resp. acid. It may range, e.g., from 0.2 mmol/mL until 200 mmol/mL or, more preferred, from 0.3 mmol/mL until 25 mmol/mL; larger or smaller concentrations may also be possible.

The invention finally also comprises the usage of the process as described above for the production of alkyl halides or benzyl halides or allyl halides or alkyl pseudo-halides or benzyl pseudo-halides or allyl pseudo-halides.

## Detailed embodiments of the invention

All chemicals used are purchased from TCI, Sigma Aldrich, and Alfa Aesar in reagent grade or better quality and are used without further purification. All solvents are distilled before usage with exception of 1,2-dichloroethane (regarding mainly chlorination with TCCA; benzylic CH-iodination with DIH is performed with distilled 1,2-dichloroethane). Analytical thin-layer chromatography (TLC) was performed on SIL G $UV_{254}$ from Macherey Nagel with a fluorescence indicator. Visualization of the TLC plate was performed by UV (254 nm), 5% phosphomolybdic acid in $CH_3CH_2OH$ or permanganate stain. Column chromatography is performed using Merck silica gel 60 (0.040-0.063 mm) or Büchi Reveleris Silica columns (0.040 mm). Flash chromatography is performed for P5 with the Reveleris X2 of Grace. $^1H$ and $^{13}C$ spectra are measured with Bruker spectrometer Avance II 200 Hz (AV 200) and Avance II 400 MHz (AV 400), Avance III HD 600 MHz (AV 600), using TMS or the solvent peak as the internal standard. NMR assignments are evaluated by Attached Proton Test (APT), Heteronuclear Single Quantum Coherence (HSQC) and Heteronuclear Multiple Bond Correlation (HMBC). $CDCl_3$ is filtered over $K_2CO_3$ to remove traces of HCl. Reaction progress is monitored by GC-MS analysis with a Quadrupol-MS HP MSD 5971 (EI) and HP 5890A GC equipped with a J & W Scientific fused silica GC column (30 m x 0.250 mm, 0.25 micron DB-5MS stationary phase: 5% phenyl and 95% methyl silicone) using He (4.6 grade) as carrier gas; T-program standard 90-250 °C (10 °C/min heating rate), injector and transfer line 250 °C. The glasses of the hood were coated with a UV foil (d-c-fix) to exclude UV light. 3-ITMH is synthesized with a purity of 94-98% determined by $^1H$ NMR. Yields for method P5 are related to the quality of 3-ITMH (94%, 0.353 mmol of pure iodine containing compound).

**Design of experiments analysis (regarding iodination)**

Exemplified calculations:

$$\text{Slope (Substrate)} = (60-28)/2 = 16$$

$$\text{Yield (Dilution/Substrate 2:1.7)} = (33+13+38+39)/4 = 31\%$$

The relative relation (Table S2) is referred to the lowest number

$$\text{Substrate} = 4.5/2.5 = 1.8$$

$$\text{Dilution/Substrate} = 14.6/6 = 2.4$$

All other parameters of the DoE-Experiments regarding iodination are presented in Fig. 16A/B-

**$^1$H NMR activation study of 3-ITMH**

3-ITMH (10.0 mg, 37.3 μmol, 1.0 equiv) and the related additives (see Fig. 11 A) are placed in the NMR tube and diluted with $CD_2Cl_2$ (600 μL). The sample is shaken or sonicated for 30 s and measured after about 0.5 h.

**DOSY experiment**

3-ITMH (10.0 mg, 37.3 μmol, 1.0 equiv) and o-nitrobenzoic acid (2.24 mg, 13.4 μmol, 0.36 equiv) are transferred to the NMR tube and diluted with $CD_2Cl_2$ (600 μL). The NMR tube is sonicated for about 30 s and then measured in a time range of 43 min.

The $^1$H DOSY spectra are carried out on a Bruker Avance III HD 600 MHz spectrometer equipped with a 5 mm broadband BBO Z-gradient probe. The temperature is set and controlled at 298 K with an air flow of 540 l $h^{-1}$. For each experiment, 16 dummy scans and 24 scans are used, with a relaxation delay of 2 s and a diffusion delay of 50 ms. The shape of the gradients are sinusoidal, with a length of 1 ms, and the strength is varied in 32 increments (2-95%) in a linear ramp. All DOSY experiments are obtained with a longitudinal eddy-current delay (LED) bipolar gradient pulse pair and two spoil gradients pulse sequence (ledbpgp2s) in the standard Bruker pulse sequence library. All experiments are processed with standard Bruker 1D and 2D DOSY software.

**General procedure P1 for the chlorination**

In a 5 mL reaction vessel $Cu(OAC)_2$•$H_2O$ (5.00 mg, 0.025 mmol, 0.02 equiv.), *N*-hydroxyphthalimide (20.4 mg, 0.125 mmol, 0.1 equiv.), $CBr_4$ (41.5 mg, 0.125 mmol, 0.1 equiv.) are diluted in $CH_2Cl_2$ (2 mL) and stirred for 0.5 min at 25 °C. Subsequently TCCA (116 mg, 0.500 mmol, 0.4 equiv.) and the substrate (1.25 mmol, 1.0 equiv.) is added and the vessel is sealed. After additional stirring for 17-22 h at 25 °C, the reaction mixture is diluted with sat. brine (10 mL), extracted with $CH_2Cl_2$ (3x10 mL), dried and concentrated under reduced pressure.

**General procedure P2 for the chlorination on gram scale without $CBr_4$**

In a 250 mL reaction vessel $Cu(OAC)_2$•$H_2O$ (0.232 g, 1.16 mmol, 0.02 equiv.), *N*-hydroxyphthalimide (0.946 g, 5.80 mmol, 0.1 equiv.) is diluted in $CH_2Cl_2$ (95 mL) and stirred for 0.5 min at 25 °C. Subsequently TCCA (5.39 g, 23.2 mmol, 0.4 equiv.) and the substrate (58.0 mmol, 1.0 equiv.) is added and the vessel is sealed. After additional stirring for 20-96 h at 25-30 °C, the reaction mixture is diluted with sat. brine (100 mL), extracted with $CH_2Cl_2$ (3x80 mL), dried and concentrated under reduced pressure.

**Benzyl chloride 4a:** (According to P1). The residue is purified by column chromatography (silica gel, 2 x 27 cm column, hexane/$Et_2O$ 9:1) to give the chloride **4a** (91.9 mg, 0.726 mmol, 58% as a colorless liquid, $R_f$ = 0.48; $^1$H

NMR (400 MHz, $CDCl_3$): $\delta$ = 7.41-7.30 (m, 5 H, $CH_{Ar}$), 4.60 (s, 2 H, $CH_2$) ppm; $^{13}C$ NMR (100 MHz, $CDCl_3$): $\delta$ = 137.6 ($C_{Ar}$), 128.9 ($CH_{Ar}$), 128.7 ($CH_{Ar}$), 128.6 ($CH_{Ar}$), 46.4 ($CH_2$) ppm.

**4-methylbenzyl chloride 6a:** (According to P1). The residue is purified by column chromatography (silica gel, 2 x 27 cm column, hexane/$Et_2O$ 19:1) to give the chloride **6a** (54.9 mg, 0.0.390 mmol, 31%) as a colorless liquid, $R_f$ = 0.51; $^1H$ NMR (400 MHz, $CDCl_3$): $\delta$ = 7.29 (d, 2 H, *J* = 8.1 Hz, $CH_{Ar}$), 7.18 (d, 2 H, *J* = 7.8 Hz, $CH_{Ar}$), 4.58 (s, 2 H, $CH_2$), 2.37 (s, 3 H, $CH_3$) ppm; $^{13}C$ NMR (100 MHz, $CDCl_3$): $\delta$ = 138.5 ($C_{Ar}$), 134.7 ($C_{Ar}$), 129.6 ($CH_{Ar}$), 128.7 ($CH_{Ar}$), 46.4 ($CH_2$), 21.3 ($CH_3$) ppm.

**1,4-Bis(chloromethyl)benzene 6b:** (According to P1). The residue is purified by column chromatography (silica gel, 2 x 27 cm column, hexane/$Et_2O$ 19:1) to give the chloride **6b** (26.6 mg, 0.152 mmol, 12%) as a colorless solid, $R_f$ = 0.35; m.p. 98-99 °C; $^1H$ NMR (400 MHz, $CDCl_3$): $\delta$ = 7.39 (s, 4 H, $CH_{Ar}$), 4.59 (s, 4 H, $CH_2$) ppm; $^{13}C$ NMR (100 MHz, $CDCl_3$): $\delta$ = 137.8 ($C_{Ar}$), 129.1 ($CH_{Ar}$), 45.8 ($CH_2$) ppm.

**3-methylbenzyl chloride 7:** (According to P1). The residue is purified by column chromatography (silica gel, 2 x 27 cm column, hexane/$Et_2O$ 19:1) to give the chloride **7** (58.4 mg, 0.415 mmol, 33%) as a colorless liquid, $R_f$ = 0.48; $^1H$ NMR (400 MHz, $CDCl_3$): $\delta$ = 7.27-7.12 (m, 4 H, $CH_{Ar}$), 4.57 (s, 2 H, $CH_2$), 2.37 (s, 3 H, $CH_3$) ppm; $^{13}C$ NMR (100 MHz, $CDCl_3$): $\delta$ = 138.6 ($C_{Ar}$), 137.5 ($C_{Ar}$), 129.5 ($CH_{Ar}$), 129.3 ($CH_{Ar}$), 128.8 ($CH_{Ar}$), 125.8 ($CH_{Ar}$), 46.5 ($CH_2$), 21.5 ($CH_3$) ppm.

**1-chloro-1-phenylethane** 8: (According to P1). The residue is purified by column chromatography (silica gel, 2 x 27 cm column, pentane) to give the chloride **8** (45.9 mg,0.327 mmol, 26%) as a colorless liquid, $R_f$ = 0.29;
According to P2: The residue is purified by distillation after 18 h to give a mixture of (2-chloroethyl)benzene (403 mg, 2.87 mmol, 5%) and chloro compound **21** (2.97 g, 21.1 mmol, 36%) as a colorless liquid, bp = 60 °C (10 mbar); yield determined by $^1H$-NMR; $^1H$ NMR (400 MHz, $CDCl_3$): $\delta$ = 7.45-7.28 (m, 5 H, $CH_{Ar}$), 5.10 (q, 1 H, *J* = 6.8 Hz, CHCl), 2.83 (d, 3 H, *J* = 6.8 Hz, $CH_3$) ppm; $^{13}C$ NMR (100 MHz, $CDCl_3$): $\delta$ = 142.8 ($C_{Ar}$), 128.6 ($CH_{Ar}$), 128.2 ($CH_{Ar}$), 126.5 ($CH_{Ar}$), 58.7 (CH), 26.5 ($CH_3$) ppm.

**1-(chloromethyl)naphthalene 9:** (According to P1). The residue is purified by column chromatography (silica gel, 2 x 27 cm column, hexane/$Et_2O$ 19:1) to give the chloride **9** (34 mg, 0.192 mmol, 15%) as a light brownish viscous oil, $R_f$ = 0.18; $^1H$ NMR (400 MHz, $CDCl_3$): $\delta$ = 8.16 (d, 1 H, *J* = 8.4 Hz, $CH_{Ar}$), 7.91-7.84 (m, 2 H, $CH_{Ar}$), 7.63-7.58 (m, 1 H, $CH_{Ar}$), 7.56-7.51 (m, 2 H, $CH_{Ar}$), 7.46-7.41 (m, 1 H, $CH_{Ar}$), 5.07 (s, 2 H, $CH_2$) ppm; $^{13}C$ NMR (100 MHz, $CDCl_3$): $\delta$ = 134.1 ($C_{Ar}$), 133.2 ($C_{Ar}$), 131.3 ($C_{Ar}$), 129.9 ($CH_{Ar}$), 129.0 ($CH_{Ar}$), 127.8 ($CH_{Ar}$), 126.9 ($CH_{Ar}$), 126.3 ($CH_{Ar}$), 125.4 ($CH_{Ar}$), 123.8 ($CH_{Ar}$), 44.7 ($CH_2$) ppm.

**2-chlorobenzyl chloride 11:** (According to P1). The residue is purified by column chromatography (silica gel, 2 x 27 cm column, hexane/$Et_2O$ 12:1) to give the chloride **11** (136 mg, 0.842 mmol, 67%) as a colorless liquid, $R_f$ = 0.48; $^1H$ NMR (400 MHz, $CDCl_3$): $\delta$ = 7.48-7.44 (m, 1 H, $CH_{Ar}$), 7.42-7.37 (m, 1 H, $CH_{Ar}$), 7.30-7.24 (m, 2 H, $CH_{Ar}$), 4.70 (s, 2 H, $CH_2$) ppm; $^{13}C$ NMR (100 MHz, $CDCl_3$): $\delta$ = 135.2 ($C_{Ar}$), 134.2 ($C_{Ar}$), 131.0 ($CH_{Ar}$), 130.1 ($CH_{Ar}$), 130.0 ($CH_{Ar}$), 127.3 ($CH_{Ar}$), 43.7 ($CH_2$) ppm.

**3-chlorobenzyl chloride 12:** (According to P1). The residue is purified by column chromatography (silica gel, 2 x 27 cm column, hexane/$Et_2O$ 12:1) to give the chloride **12** (110 mg, 0.684 mmol, 55%) as a colorless liquid, $R_f$ = 0.46; $^1H$ NMR (400 MHz, $CDCl_3$): $\delta$ = 7.39 (bs, 1 H, $CH_{Ar}$), 7.33-7.24 (m, 3 H, $CH_{Ar}$), 4.54 (s, 2 H, $CH_2$) ppm; $^{13}C$ NMR (100 MHz, $CDCl_3$): $\delta$ = 139.4 ($C_{Ar}$), 134.6 ($C_{Ar}$), 130.1 ($CH_{Ar}$), 128.8 ($CH_{Ar}$), 128.7 ($CH_{Ar}$), 126.8 ($CH_{Ar}$), 45.4 ($CH_2$) ppm.

**4-chlorobenzyl chloride 13:** (According to P1). The residue is purified by column chromatography (silica gel, 2 x 27 cm column, hexane/$Et_2O$ 20:1) to give the chloride **13** (147 mg, 0.912 mmol, 73%) as a colorless solid, $R_f$ = 0.37; m.p. 28-29 °C; $^1H$ NMR (400 MHz, $CDCl_3$): $\delta$ = 7.35-7.31 (m, 4 H, $CH_{Ar}$), 4.55 (s, 2 H, $CH_2$) ppm; $^{13}C$ NMR (100 MHz, $CDCl_3$): $\delta$ = 136.1 ($C_{Ar}$), 134.4 ($C_{Ar}$), 130.1 ($CH_{Ar}$), 129.1 ($CH_{Ar}$), 45.5 ($CH_2$) ppm.

**Methyl 2-(chloromethyl)benzoate 14:** (According to P1). The residue is purified by column chromatography (silica gel, 2 x 27 cm column, hexane/$Et_2O$ 9:1) to give the chloride **14** (141 mg, 0.764 mmol, 61%) as a colorless liquid, $R_f$ = 0.20; $^1H$ NMR (400 MHz, $CDCl_3$): $\delta$ = 7.98 (d, 1 *H, J* = 7.8 Hz, $CH_{Ar}$), 7.60-7.50 (m, 2 H, $CH_{Ar}$), 7.43-7.37 (m, 1 H, $CH_{Ar}$), 5.05 (s, 2 H, $CH_2$), 3.94 (s, 3 H, $CH_3$) ppm; $^{13}C$ NMR (100 MHz, $CDCl_3$): $\delta$ = 167.2 (CO), 138.9 ($C_{Ar}$), 132.7 ($C_{Ar}$), 131.2 ($CH_{Ar}$), 131.0 ($CH_{Ar}$), 129.2 ($CH_{Ar}$), 128.5 ($CH_{Ar}$), 52.4 ($CH_2$), 44.6 ($CH_3$) ppm.

**Methyl 3-(chloromethyl)benzoate 15:** (According to P1). The residue is purified by column chromatography (silica gel, 2 x 27 cm column, hexane/$Et_2O$ 9:1) to give the chloride **15** (115 mg, 0.625 mmol, 50%) as a colorless liquid, $R_f$ = 0.10; $^1H$ NMR (400 MHz, $CDCl_3$): $\delta$ = 8.06 (s, 1 H, $CH_{Ar}$), 7.99 (d, 1 *H, J* = 7.8 Hz, $CH_{Ar}$), 7.58 (d, 1 H, *J* = 7.6 Hz, $CH_{Ar}$), 7.43 (t, 1 H, *J* = 7.7 Hz, $CH_{Ar}$), 4.61 (s, 2 H, $CH_2$), 3.92 (s, 3 H, $CH_3$) ppm; $^{13}C$ NMR (100 MHz, $CDCl_3$): $\delta$ = 166.6 (CO), 138.0 ($C_{Ar}$), 133.1 ($C_{Ar}$), 130.8 ($CH_{Ar}$), 129.8 ($CH_{Ar}$), 129.6 ($CH_{Ar}$), 129.0 ($CH_{Ar}$), 52.3 ($CH_2$), 45.6 ($CH_3$) ppm.

**Methyl 4-(chloromethyl)benzoate 16:** (According to P1). The residue is purified by column chromatography (silica gel, 2 x 27 cm column, hexane/$Et_2O$ 9:1) to give the chloride **16** (196 mg, 1.06 mmol, 85%) as a colorless solid, $R_f$ = 0.12; m.p. 36-37 °C; $^1H$ NMR (400 MHz, $CDCl_3$): $\delta$ = 8.03 (d, 2 H, *J* = 8.2 Hz, $CH_{Ar}$), 7.46 (d, 2 H, *J* = 8.2 Hz, $CH_{Ar}$), 4.61 (s, 2 H, $CH_2$), 3.92 (s, 3 H, $CH_3$) ppm; $^{13}C$-NMR (100 MHz, $CDCl_3$): $\delta$ = 166.7 (CO), 142.3 ($C_{Ar}$), 130.2 ($C_{Ar}$), 130.1 ($CH_{Ar}$), 128.6 ($CH_{Ar}$), 52.3 ($CH_2$), 45.5 ($CH_3$) ppm.

**4-(chloromethyl)benzonitrile 17:** (According to P1). The residue is purified by column chromatography (silica

gel, 2 x 27 cm column, hexane/$Et_2O$ 4:1) to give the chloride **17** (112 mg, 0.741 mmol, 59%)/(160 mg, 1.06 mmol, 70% (two equiv. of substrate used; yield related to TCCA)) as a colorless solid, $R_f$ = 0.22; m.p, 80-81 °C; $^1$H NMR (400 MHz, $CDCl_3$): $\delta$ = 7.66 (d, 2 H, *J* = 8.3 Hz, $CH_{Ar}$), 7.51 (d, 2 H, *J* = 8.3 Hz, $CH_{Ar}$), 4.60 (s, 2 H, $CH_2$) ppm; $^{13}$C NMR (100 MHz, $CDCl_3$): $\delta$ = 142.5 ($C_{Ar}$), 132.7 ($CH_{Ar}$), 129.3 ($CH_{Ar}$), 118.5 ($C_{Ar}$), 112.4 ($C_{Ar}$), 45.0 ($CH_2$) ppm.

**Chlorocyclopentane 20:** (According to P2). The residue is purified by distillation to give the chloro compound **20** (1.86 g, 17.7 mmol, 31%) as a colorless liquid; $^1$H NMR (400 MHz, $CDCl_3$): $\delta$ = 4.44-4.33 (m, 1 H, CHCl), 2.05-1.81 (m, 6 H, $CH_2$), 1.73-1.58 (m, 2 H, $CH_2$) ppm; $^{13}$C NMR (50 MHz, $CDCl_3$): $\delta$ = 62.3 (CHCl), 37.2 ($CH_2$), 23.1 ($CH_2$) ppm.

**Chlorocyclohexane 21:** (According to P2). The residue is purified by distillation to give the chloro compound **21** (2.60 g, 22.2 mmol, 38%) as a colorless liquid, bp = 142 °C; $^1$H NMR (200 MHz, $CDCl_3$): $\delta$ = 4.10-3.94 (m, 1 H, CHCl), 2.13-1.93 (m, 2 H, $CH_2$), 1.90-1.20 (m, 8 H, $CH_2$) ppm; $^{13}$C NMR (50 MHz, $CDCl_3$): $\delta$ = 60.5 (CHCl), 36.8 ($CH_2$), 25.3 ($CH_2$), 25.0 ($CH_2$) ppm.

**Chlorocyclooctane 22:** (According to P2). The residue is purified by distillation to give the chloro compound **22** (2.01 g, 13.8 mmol, 24%) as a colorless liquid, bp = 80 °C (10 mbar); $^1$H NMR (400 MHz, $CDCl_3$): $\delta$ = 4.23 (sept, 1 H, CHCl), 2.16-2.06 (m, 2 H, $CH_2$), 2.05-1.91 (m, 2 H, $CH_2$), 1.83-1.68 (m, 2 H, $CH_2$), 1.65-1.42 (m, 8 H, $CH_2$) ppm; $^{13}$C NMR (50 MHz, $CDCl_3$): $\delta$ = 63.7 (CHCl), 35.3 ($CH_2$), 27.6 ($CH_2$), 25.1 ($CH_2$), 23.7 ($CH_2$) ppm.

**General procedure for the iodination (P3):** In a 5 mL reaction vessel *N*-hydroxyphthalimide (10.2 mg, 0.0625 mmol, 0.17 equiv.), o-nitro benzoic acid (22.7 mg, 0.136 mmol, 0.36 equiv.) are diluted in $CH_2Cl_2$ (2 mL) and stirred for 1 min at 25 °C. Subsequently DIH (143 mg, 0.375 mmol, 1 equiv.) and the substrate (2.50 mmol, 6.8 equiv.) is added and the vessel is sealed. After an additional stirring for 3 h at 25 °C, the reaction mixture is diluted with EtOAc (20 mL), washed with saturated $NaHCO_3$ (10 mL), $Na_2S_2O_3$ (5%, 10 mL), dried and concentrated under reduced pressure.

**General procedure for the iodination with DIH (P4):** In a 5 or 10 mL reaction vessel *N*-hydroxyphthalimide (10.2 mg, 0.0625 mmol, 0.17 equiv), o-nitrobenzoic acid (22.7 mg, 0.136 mmol, 0.36 equiv) is diluted in $CH_2Cl_2$ (2 mL) and stirred for 1 min at 25 °C. Subsequently DIH (143 mg, 0.375 mmol, 1.0 equiv) and the substrate (2.50 mmol, 6.7 equiv) is added and the vessel is sealed. After an additional stirring for 3 h at 25-27 °C, the reaction mixture is diluted with EtOAc (20 mL), washed with sat. $NaHCO_3$ (10 mL), $Na_2S_2O_3$ (5%, 10 mL), dried over $Na_2SO_4$ and concentrated under reduced pressure.

**General procedure for the iodination with 3-ITMH (P5):** In a 10 mL reaction vessel *N*-hydroxyphthalimide (10.2 mg, 0.0625 mmol, 0.17 equiv), o-nitrobenzoic acid (22.7 mg, 0.136 mmol, 0.36 equiv) is diluted in $CH_2Cl_2$ (2 mL) and stirred for 1 min at 25 °C. Subsequently 3-ITMH (94%, 100 mg, 0.375 mmol, 1.0 equiv) and the substrate (2.50 mmol, 6.7 equiv) is added and the vessel is sealed. After an additional stirring for 3 h at 25-27 °C, the reaction mixture is diluted with $CH_2Cl_2$ (20 mL), washed with $Na_2S_2O_3$ (5%, 10 mL) and the aqueous phase extracted with $CH_2Cl_2$ (2 x 10 mL). The combined organic layers are dried over $Na_2SO_4$ and concentrated under reduced pressure.

**1-ITMH:** According to the state of the art a mixture of 3,5,5-trimethylhydantoin (860 mg, 6.05 mmol, 1 equiv), $PhI(OAc)_2$ (1.18 g, 3.65 mmol), $I_2$ (1.01 g, 3.95 mmol), and cyclohexane (17.3 mL) is stirred at rt for 40 h and then cooled to 0 °C, whereat stirring is being continued for an additional hour. The residue is filtered (glass frit D4), washed with $CH_2Cl_2$ (2 x 10 mL) and dried over paraffin to afford **1** (1.57 g, 5.86 mmol, 96%) as a pale pink solid; $^1$H NMR (400 MHz, $CDCl_3$): $\delta$ = 3.09 (s, 3 H), 1.25 (s, 6 H) ppm.

**3-ITMH:** 1,5,5-trimethylhydantoin (1.91 g, 13.4 mmol, 1 equiv), $PhI(OAc)_2$ (2.81 g, 8.70 mmol, 0.65 equiv) and $I_2$ (2.41 g, 9.50 mmol, 0.71 equiv) is diluted in $CH_3CN$ (23 mL). After stirring for 18 h the solvent is removed under reduced pressure, the residue diluted in $CH_3CN$ (23 mL) and stirred for 1 h. The solvent is removed under reduced pressure, treated with $CH_2Cl_2$ (40 mL) and stirred for 0.5 h. Filtration (glass frit D4), washing with $CH_2Cl_2$ (2 x 10 mL) and drying over paraffin affords 3-ITMH (3.13 g, 11.6 mmol, 85%) as a pale pink solid; $^1$H NMR (400 MHz, $CDCl_3$): $\delta$ = 2.95 (s, 3 H), 1.39 (s, 6 H) ppm; $^{13}$C NMR (100 MHz, $CDCl_3$): $\delta$ = 178.7, 154.4, 64.2, 26.1, 22.9 ppm; $^1$H NMR (400 MHz, DMSO-$d_6$): $\delta$ = 2.77 (s, 3 H), 1.22 (s, 6 H) ppm; $^{13}$C NMR (100 MHz, DMSO-$d_6$): $\delta$ = 179.9, 155.9, 62.6, 25.1, 22.1 ppm.

**4-(Trifluoromethyl)benzyl iodide 28:** (According to P3, 19 h). The residue is purified by column chromatography (silica gel, 2 x 27 cm column, hexane/EtOAc 9:1) to give the iodide **28** (79.2 mg, 0.277 mmol, 37%) as a colorless solid, $R_f$ = 0.36; m.p. 44 °C; $^1$H NMR (400 MHz, $CDCl_3$): δ = 7.56 (d, 2 H, *J* = 8.1 Hz, $CH_{Ar}$), 7.48 (m, 2 H, *J* = 8.1 Hz, $CH_{Ar}$), 4.46 (s, 2 H, $CH_2$) ppm; $^{13}$C NMR (100 MHz, $CDCl_3$): δ = 143.5 (d, *J* = 1.3 Hz), 130.1 (q, *J* = 33.6 Hz), 129.2, 126.0 (q, *J* = 3.8 Hz), 124.1 (q, *J* = 273.1 Hz), 3.31 ($CH_2$) ppm.

**4-(Trifluoromethyl)benzyl iodide 28:** (According to P4, 19 h). The residue is purified by column chromatography (silica gel, hexane/EtOAc 9:1) to give the iodide **28** (79.2 mg, 0.277 mmol, 37%) as a colorless solid, $R_f$ = 0.36; m.p. 44 °C; $^1$H NMR (400 MHz, $CDCl_3$): $\delta$ = 7.56 (d, 2 H, *J* = 8.1 Hz), 7.48 (m, 2 H, *J* = 8.1 Hz), 4.46 (s, 2 H) ppm; $^{13}$C NMR (100 MHz, $CDCl_3$): $\delta$ = 143.5 (d, *J* = 1.3 Hz), 130.1 (q, *J* = 33.6 Hz), 129.2, 126.0 (q, *J* = 3.8 Hz), 124.1 (q, *J* = 273.1 Hz), 3.31 ppm.

**4-(Trifluoromethyl)benzyl iodide 28:** (According to P5). The residue is purified by flash column chromatography (silica gel, hexane) giving **28** (68.1 mg, 0.238 mmol, 68%).

**4-Nitrobenzyl iodide 29:** (According to **P3,** 22 h). The residue is purified by column chromatography (silica gel, 2 x 27 cm column, hexane/EtOAc 9:1) to give the iodide **29** (121 mg, 0.459 mmol, 61%) as a colorless solid, $R_f$ =

0.24; m.p. 127.4 °C; $^{1}$H NMR (400 MHz, $CDCl_3$): δ = 8.16 (d, 2 H, *J* = 8.7 Hz, $CH_{Ar}$), 7.52 (m, 2 H, *J* = 8.7 Hz, $CH_{Ar}$), 4.48 (s, 2 H, $CH_2$) ppm; $^{13}$C NMR (100 MHz, $CDCl_3$): δ = 147.4, 146.9, 129.7, 124.3, 2.2 ($CH_2$) ppm.

**4-Nitrobenzyl iodide 29:** (According to P4, 22 h). The residue is purified by column chromatography (silica gel, hexane/EtOAc 9:1) to give the iodide **29** (121 mg, 0.459 mmol, 61%) as a colorless solid, $R_f$ = 0.24; m.p. 127.4 °C (Lit. 125-126 °C); $^{1}$H NMR (400 MHz, $CDCl_3$): δ = 8.16 (d, 2 H, *J* = 8.7 Hz), 7.52 (m, 2 H, *J* = 8.7 Hz), 4.48 (s, 2 H,) ppm; $^{13}$C NMR (100 MHz, $CDCl_3$): δ = 147.4, 146.9, 129.7, 124.3, 2.2 ppm.

**4-Nitrobenzyl iodide 29:** (According to P5). The residue is purified by flash column chromatography (silica gel, hexane/EtOAc 24:1) giving **29** (66.5 mg, 0.253 mmol, 72%).

**Methyl 4-(iodomethyl)benzoate 30:** (According to P3). The residue is purified by column chromatography (silica gel, 2 x 27 cm column, hexane/$Et_2O$ 9:1) to give methyl 4-(iodomethyl)benzoate (134 mg, 0.485 mmol, 65%) as a colorless solid, $R_f$ = 0.14; m.p. 72-73 °C; $^{1}$H NMR (400 MHz, $CDCl_3$): δ = 7.98-7.94 (m, 2 H, $CH_{Ar}$), 7.45-7.41 (m, 2 H, $CH_{Ar}$), 4.46 (s, 2 H, $CH_2$), 3.91 (s, 3 H, $CH_3$) ppm; $^{13}$C-NMR (100 MHz, $CDCl_3$): δ = 166.7 (CO), 144.5 ($C_{Ar}$), 130.3 ($C_{Ar}$), 129.7 ($CH_{Ar}$), 128.9 ($CH_{Ar}$), 52.3 ($CH_3$), 4.0 ($CH_2$) ppm.

**Methyl 4-(iodomethyl)benzoate 30:** (According to P4). The residue is purified by column chromatography (silica gel, hexane/$Et_2O$ 9:1) to give the iodide **30** (134 mg, 0.485 mmol, 65%) as a colorless solid, $R_f$ = 0.14; m.p. 72-73 °C (Lit. 73-74 °C); $^{1}$H NMR (400 MHz, $CDCl_3$): δ = 7.98-7.94 (m, 2 H), 7.45-7.41 (m, 2 H), 4.46 (s, 2 H), 3.91 (s, 3 H) ppm; $^{13}$C-NMR (100 MHz, $CDCl_3$): δ = 166.7, 144.5, 130.3, 129.7, 128.9, 52.3, 4.0 ppm.

**Methyl 4-(iodomethyl)benzoate 30:** (According to P5). The residue is purified by flash column chromatography (silica gel, hexane/EtOAc 24:1) giving **30** (62.9 mg, 0.228 mmol, 65%).

**2-chlorobenzyl iodide 31:** (According to P3). The residue is purified by column chromatography (silica gel, 2 x 27 cm column, hexane) to give the iodide **31** (127.0 mg, 0.503 mmol, 67%) as a colorless oil, $R_f$ = 0.26; $^{1}$H NMR (400 MHz, $CDCl_3$): δ = 7.44-7.38 (m, 1 H, $CH_{Ar}$), 7.37-7.32 (m, 1 H, $CH_{Ar}$), 7.24-7.18 (m, 2 H, $CH_{Ar}$), 4.53 (s, 2 H, $CH_2$) ppm; $^{13}$C NMR (100 MHz, $CDCl_3$): δ = 136.9, 134.0, 130.8, 130.3, 129.6, 127.5, 2.5 ($CH_2$) ppm.

**2-chlorobenzyl iodide 31:** (According to P4). The residue is purified by column chromatography (silica gel, hexane) to give the iodide **31** (127.0 mg, 0.503 mmol, 67%) as a colorless oil, $R_f$ = 0.26; $^{1}$H NMR (400 MHz, $CDCl_3$): δ = 7.44-7.38 (m, 1 H), 7.37-7.32 (m, 1 H), 7.24-7.18 (m, 2 H), 4.53 (s, 2 H) ppm; $^{13}$C NMR (100 MHz, $CDCl_3$): δ = 136.9, 134.0, 130.8, 130.3, 129.6, 127.5, 2.5 ppm.

**2-chlorobenzyl iodide 31:** (According to P5). The residue is purified by flash column chromatography (silica gel, hexane) giving **31** (56.6 mg, 0.224 mmol, 64%).

**3-chlorobenzyl iodide 32:** (According to P3, 23 h). The residue is purified by column chromatography (silica gel, 2 x 27 cm column, hexane) to give the iodide **32** (93.0 mg, 0.368 mmol, 49%) as a colorless oil, $R_f$ = 0.29; $^{1}$H NMR (400 MHz, $CDCl_3$): δ = 7.37 (q, 1 H, *J* = 1.4 Hz, $CH_{Ar}$), 7.27-7.20 (m, 3 H, $CH_{Ar}$), 4.39 (s, 2 H, $CH_2$) ppm; $^{13}$C NMR (100 MHz, $CDCl_3$): δ = 141.4, 134.6, 130.2, 129.0, 128.2, 127.1, 3.74 ($CH_2$) ppm.

**4-(iodomethyl)benzonitrile 34:** (According to P3). The residue is purified by column chromatography (silica gel, 2 x 27 cm column, hexane/EtOAc 9:1) to give the iodide **34** (64.6 mg, 0.266 mmol, 35%) as a colorless solid, $R_f$ = 0.21; m.p. 140-142 °C; $^{1}$H NMR (400 MHz, $CDCl_3$): δ = 7.59 (d, 2 H, *J* = 8.4 Hz, $CH_{Ar}$), 7.46 (d, 2 H, *J* = 8.3 Hz, $CH_{Ar}$), 4.44 (s, 2 H, $CH_2$) ppm; $^{13}$C NMR (100 MHz, $CDCl_3$): δ = 144.8, 132.7, 129.6, 118.6, 111.7, 2.9 ($CH_2$) ppm.

**4-(iodomethyl)benzonitrile 34:** (According to P4). The residue is purified by column chromatography (silica gel, hexane/EtOAc 9:1) to give the iodide **34** (64.6 mg, 0.266 mmol, 35%) as a colorless solid, $R_f$ = 0.21; m.p. 140-142 °C (Lit. 140-144 °C); $^{1}$H NMR (400 MHz, $CDCl_3$): δ = 7.59 (d, 2 H, *J*= 8.4 Hz), 7.46 (d, 2 H, *J* = 8.3 Hz), 4.44 (s, 2 H, $CH_2$) ppm; $^{13}$C NMR (100 MHz, $CDCl_3$): δ = 144.8, 132.7, 129.6, 118.6, 111.7, 2.9 ppm.

**4-(iodomethyl)benzonitrile 34:** (According to P5).The residue is purified by flash column chromatography (silica gel, hexane to hexane/EtOAc 19:1) giving **34** (43.1 g, 0.177 mmol, 50%).

**Benzyl iodide 35:** (According to P3). The residue is purified by column chromatography (silica gel, 2 x 27 cm column, hexane) to give the iodide **34** (99.0 mg, 0.454 mmol, 60%) as a colorless solid, $R_f$ = 0.24; m.p. 24-25 °C $^{1}$H NMR (400 MHz, $CDCl_3$): δ = 7.40-7.36 (m, 2 H, $CH_{Ar}$), 7.32-7.21 (m, 3 H, $CH_{Ar}$), 4.46 (s, 2 H, $CH_2$) ppm; $^{13}$C NMR (100 MHz, $CDCl_3$): δ = 139.5, 129.0, 128.9, 128.0, 5.8 ($CH_2$) ppm.

**Benzyl iodide 35:** (According to P4). The residue is purified by column chromatography (silica gel, hexane) to give the iodide **35** (99.0 mg, 0.454 mmol, 60%) as a colorless solid below r.t. (22 to 23 °C), $R_f$ = 0.24; m.p. at r.t. (Lit. 23-24 °C); $^{1}$H NMR (400 MHz, $CDCl_3$): δ = 7.40-7.36 (m, 2 H), 7.32-7.21 (m, 3 H), 4.46 (s, 2 H) ppm; $^{13}$C NMR (100 MHz, $CDCl_3$): δ = 139.5, 129.0, 128.9, 128.0, 5.8 ppm.

**Benzyl iodide 35:** (According to P5). The residue is purified by flash column chromatography (silica gel, hexane), giving **35** (51.2 mg, 0.235 mmol, 67%).

**Methyl 2-(iodomethyl)benzoate 36:** (According to P4). The residue is purified by column chromatography (silica gel, hexane/$Et_2O$ 9:1) to give the iodide **36** (99.6 mg, 0.361 mmol, 48%) as a colorless solid, $R_f$ = 0.27; m.p. 57-58 °C; $^{1}$H NMR (400 MHz, $CDCl_3$): δ = 7.95 (dd, 1 H, *J = 7.7, 1.2* Hz, $CHCCO_2CH_3$), 7.47-7.40 (m, 2 H, $CH_2CCHCHCH$), 7.35-7.30 (m, 1 H, $CHCHCCH_2$), 4.93 (s, 2 H, $CH_2$), 3.95 (s, 3 H, $CH_3$) ppm; $^{13}$C NMR (100 MHz, $CDCl_3$): δ = 167.2 (CO), 141.4 ($CCH_2$), 132.8 ($CH_{Ar}$), 131.7 (CHCCO), 131.4 ($CH_{Ar}$), 128.4 (CCO), 128.1 ($CHCHCCH_2$), 52.4 ($CH_3$), 4.1

($CH_2$) ppm; HRMS (ESI) calculated for $C_9H_9INaO_2$ ([M+Na]$^+$): 298.9545. Found: 298.9551; elemental analysis calcd (%) for $C_9H_9IO_2$ (276.1): C 39.16, H 3.29. Found C 39.41, H 3.25.

**Methyl 2-(iodomethyl)benzoate 36:** (According to P5). The residue is purified by flash column chromatography (silica gel, hexane/EtOAc 24:1) giving **35** (53.5 mg, 0.194 mmol, 55%).

**Methyl 3-(iodomethyl)benzoate 37:** (According to P4). The residue is purified by column chromatography (silica gel, hexane/$Et_2O$ 9:1) to give the iodide **37** (106 mg, 0.384 mmol, 51%) as a colorless solid, $R_f$ = 0.22; m.p. 51-52 °C (Lit. 52-53 °C); $^1$H NMR (400 MHz, $CDCl_3$): $\delta$ = 8.04 (t, 1 H, $J$ = 1.8 Hz, $CH_3O_2CCCHCCH_2I$), 7.91 (dt, 1 H, $J$ = 7.8, 1.4 Hz, $CHCHCCO_2CH_3$), 7.56 (dt, 1 H, $J$ = 7.8, 1.6 Hz, $CHCHCCH_2I$), 7.38 (t, 1 H, $J$ = 7.7 Hz, CHCHCH), 4.47 (s, 2 H, $CH_2$), 3.92 (s, 3 H, $CH_3$) ppm; $^{13}$C NMR (100 MHz, $CDCl_3$): $\delta$ = 166.6 (CO), 139.9 ($CCH_2$), 133.3 ($CHCHCCH_2$), 130.9 (CCO), 129.8 (CCHC), 129.1 (COCCHCH), 52.4 ($CH_3$), 4.3 ($CH_2$) ppm; HRMS (ESI) calculated for $C_9H_9INaO_2$ ([M+Na]$^+$): 298.9545. Found: 298.9545; elemental analysis calcd (%) for $C_9H_9O_2$ (276.1): C 39.16, H 3.29. Found: C 39.19, H 3.12.

**Methyl 3-(iodomethyl)benzoate 37:** (According to P5) The residue is purified by flash column chromatography (silica gel, hexane/EtOAc 24:1) giving **37** (53.2 mg, 0.192 mmol, 55%).

**4-(iodoethyl)benzonitrile 38:** (According to P4). The residue is purified by column chromatography (silica gel, hexane/EtOAc 9:1) to give the iodide **38** (133 mg, 0.517 mmol, 69%) as a pale yellow oil, $R_f$ = 0.18; $^1$H NMR (400 MHz, $CDCl_3$): $\delta$ = 7.60 (d, 2 H, $J$ = 8.4 Hz, $CH_{Ar}$), 7.52 (d, 2 H, $J$ = 8.4 Hz, $CH_{Ar}$), 5.33 (q, 1 H, $J$ = 7.1 Hz, CH), 2.20 (d, 3 H, $J$ = 7.1 Hz, $CH_3$) ppm; $^{13}$C NMR (100 MHz, $CDCl_3$): $\delta$ =150.5 (CCHI), 132.7 ($CH_{Ar}$), 127.5 ($CH_{Ar}$), 118.7 (CN), 111.6 (CCN), 28.3 ($CH_3$), 22.5 (CH) ppm; IR (ATR): 2971.0, 2227.3, 1604.6, 1503.2, 835.0, 570.6 cm$^{-1}$; HRMS (ESI) calculated for $C_9H_8INNa$ ([M+Na]$^+$): 279.9594. Found: 279.9593; elemental analysis calcd (%) for $C_9H_8IN$ (257.1): C 42.05, H 3.14, N 5.45. Found: C 42.09, H 3.18, N 5.50.

**4-(iodoethyl)benzonitrile 38:** (According to P5, but without acid). The residue is purified by flash column chromatography (silica gel, hexane/EtOAc 24:1) giving **38** (53.5 g, 0.208 mmol, 59%).

**4-Fluorobenzyl iodide 39:** (According to P4). The residue is purified by column chromatography (silica gel, hexane) to give the iodide **39** 70.6 mg, 0.299 mmol, 40%) as a colorless solid, $R_f$ = 0.23; m.p. 34 °C (Lit. 34-35 °C); $^1$H NMR (400 MHz, $CDCl_3$): $\delta$ = 7.38-7.32 (m, 2 H, $CH_{Ar}$), 7.02-6.95 (m, 2 H, $CH_{Ar}$), 4.44 (s, 2 H, $CH_2$) ppm; $^{13}$C NMR (100 MHz, $CDCl_3$): $\delta$ = 162.3 (d, $J$ = 247.6 Hz, CF), 135.3 (d, $J$ = 3.4 Hz, $CCH_2$), 130.6 (d, $J$ = 8.3 Hz, $CHCCH_2I$), 115.9 (d, $J$ = 21.7 Hz, CHCF), 4.7 ppm.

**4-Fluorobenzyl iodide 39:** (According to P5). The residue is purified by flash column chromatography (silica gel, hexane) giving **39** (56.7 mg, 0.240 mmol, 68%).

**3,5,5-trimethylhydantoin:** 5,5-dimethylhydantoin (1.00 g, 7.80 mmol, 1 equiv) and $K_2CO_3$ (50%, 1.10 g, 7.80 mmol, 1 equiv) in EtOH (6 mL) is stirred for 0.5 h. MeI (1.11 g, 7.80 mmol, 1 equiv) is added dropwise and the mixture stirred for additional 2 h at r.t.. Then the temperature is increased to 60 °C and the mixture stirred for 3 h. The solvent is evaporated under reduced pressure and the residue solved in $H_2O$ (4 mL). Extraction with $CHCl_3$ (3 x 4 mL), drying over $Na_2SO_4$ and evaporation of the solvent at reduced pressure affords 3,5,5-trimethylhydantoin (876 mg, 6.15 mmol, 79%) as a white solid. $^1$H NMR (400 MHz, $CDCl_3$): $\delta$ = 5.45 (bs, 1 H), 3.02 (s, 3 H), 1.44 (s, 6 H) ppm; $^{13}$C NMR (100 MHz, $CDCl_3$): $\delta$ = 177.6, 156.9, 59.1, 25.2, 24.8 ppm.

**General procedure and parameters for the DoE-Experiments and the Screening for radical initiators**

Reagents and catalysts **43, 44, 45** were purchased from TCI or Sigma Aldrich in reagent grade or better quality and used without further purification: $Cu(OAc)_2$. $H_2O$ > 99%, NHPI > 99% (TCI), TCCA ≤ 100%. All solvents were distilled before use. $^1$H-NMR spectra were measured at 25 ° C with a 400 MHz spectrometer using the solvent peak ($CHCl_3$ : 7.26 ppm) as the internal standard.

**4-Methoxy-*N*-hydroxyphthalimide, 40:** The compound was synthesized according to the method of Sugamoto *et al.* which is well known to the person skilled in the art. **40** (100 mg, 0.518 mmol, 37%) is obtained as a light yellowish solid; m.p. 209-210 °C (Lit. 213 °C); $^1$H NMR (400 MHz, DMSO-$d_6$): $\delta$ = 10.70 (bs, 1 H), 7.76 (d, 1 H, $J$ = 8.3 Hz), 7.36 (d, 1 H, $J$ = 2.4 Hz), 7.29 (dd, 1 H, $J$ = 8.3, 2.3 Hz), 3.91 (s, 3 H) ppm; $^{13}$C NMR (100 MHz, DMSO-$d_6$): $\delta$ = 164.4, 163.9, 163.8, 131.3, 125.0, 120.3, 119.3, 108.7, 56.2 ppm.

**4-Methyl-*N*-hydroxyphthalimide, 41:** The compound was synthesized according to the method of Sugamoto *et al.* which is well known to the person skilled in the art. **41** (121 mg, 0.685 mmol, 49%) is obtained as a light yellowish solid; m.p. 198 °C (Lit. 202 °C); $^1$H NMR (400 MHz, DMSO-$d_6$): $\delta$ = 10.75 (bs, 1 H), 7.73-7.68 (m, 1 H), 7.67-7.60 (m, 2 H), 2.46 (s, 3 H) ppm; $^{13}$C NMR (100 MHz, DMSO-$d_6$): $\delta$ = 164.2, 145.5, 134.7, 129.0, 126.0, 123.5, 122.9, 21.4 ppm.

**3-Methyl-*N*-hydroxyphthalimide, 42:** The compound was synthesized according to the method of Sugamoto *et al.* which is well known to the person skilled in the art. **42** (169 mg, 0.955 mmol, 68%) is obtained as a white solid; m.p. 221 °C (Lit. 221 °C); $^1$H NMR (400 MHz, DMSO-$d_6$): $\delta$ = 10.70 (bs, 1 H), 7.70-7.55 (m, 3 H), 2.58 (s, 3 H) ppm; $^{13}$C NMR (100 MHz, DMSO-$d_6$): $\delta$ = 164.8, 163.9, 137.0, 136.6, 133.9, 129.1, 125.3, 120.5, 17.0 ppm.

**4-Methoxyphthalic anhydride:** The compound was synthesized according to the method of Tilvawala *et al.*

which is well known to the person skilled in the art. The 4-methoxyphthalic anhydride (514 mg, 2.89 mmol, 90%) is obtained as a colorless solid; m.p. 89-90 °C (Lit.: 88-90 °C); $^{1}H$ NMR (400 MHz, DMSO-$d_6$): δ = 7.99 (d, 1 H, *J* = 8.5 Hz), 7.58 (d, 1 H, *J = 2.3* Hz), 7.49 (dd, 1 H, *J* = 8.4, 2.3 Hz), 3.97 (s, 3 H) ppm; $^{13}C$ NMR (100 MHz, DMSO-$d_6$): δ = 165.7, 163.1, 162.7, 134.0, 127.2, 122.8, 122.7, 109.3, 56.7 ppm.

**General procedure P6 for bromination**

In a 10 mL reaction vessel $Cu(OAc)_2•H_2O$ (5.00 mg, 0.025 mmol, 0.02 equiv.), *N*-hydroxyphthalimide (20.4 mg, 0.125 mmol, 0.1 equiv.), $CBr_4$ (41.5 mg, 0.125 mmol, 0.1 equiv.) are diluted in $CH_2Cl_2$ (2 mL) and stirred for 0.5 min at 25 °C. Subsequently 1,3-dibromo-5,5-dimethylhydantoin **8a** (200 mg, 0.700 mmol, 0.6 equiv.) and the substrate (1.25 mmol, 1.0 equiv.) is added and the vessel is sealed. After additional stirring for 18-22 h at 25-27 °C, DMT (0.625 M in $CH_2Cl_2$, 200 μL, 0.1 equiv.) was added to the reaction mixture and stirred for two to five minutes. About 100 μL of the reaction mixture was used and the solvent was evaporated in a nitrogen stream. The residue was dissolved in $CD_2Cl_2$ (0.6 mL) and investigated, revealing the results shown in scheme 8B.

**Computations at the B3LYP-D3/6-31G(d,p)/CPCM level of theory**

**(solvent: $CH_2Cl_2$)**

All calculations based on Kohn-Sham density functional theory (DFT) are performed by means of the program package Gaussian09. Geometry optimizations are performed with the B3LYP. Empirical atom dispersion corrections by Grimme (D3) are used to treat non-covalent interactions. Frequency analysis is performed at the same level of theory to confirm that the optimized structures are local minima or transition states, and also to obtain the thermodynamic corrections to Gibbs free energy. Copper is treated by cc-PVDZ-DK all-electron basis set, while all remaining elements are represented by 6-31 G(d,p). The intrinsic reaction coordinate (IRC) calculations are performed to ensure that the obtained transition states connect the right reactants and products. Solution-phase (CPCM) single-point energy calculations are conducted on the basis of the gas-phase-optimized structures using dichloromethane as solvent at 298 K and 1 atm.

**Computations at the M06-2X/cc-pVTZ/CPCM level of theory (solvent: $CH_2Cl_2$)**

All structures are optimized at the M06-2X/cc-pVTZ/CPCM (solvent: $CH_2Cl_2$) level of theory utilizing the Gaussian09 program package. The computed structures are characterized as minima on the potential energy hypersurface by vibrational frequency computations (with minima having only real frequencies) that also provide thermal corrections and zero-point vibrational energies (ZPVEs).

**Description of the drawings**

Fig. 1: Table 1 - Reaction conditions (and reaction scheme) for the side-chain chlorination of toluene. ([a] Determined by GC-MS; [b] GC-MS ratios; [c] TCCA (1.0 equiv.); [d] After 16 h MeOH (2 equiv.) added; [e] TBHP (0.1 equiv.) added; [f] After 40 h additional TCCA (0.4 equiv.) added with no further conversion; 9 $Co(OAC)_2•4H_2O$ (0.01 equiv.); [h] $Co(OAC)_2•4H_2O$ (0.05 equiv.); [l] $Co(OAC)_2•4H_2O$ (0.1 equiv.); [j] without $Co(OAC)_2•4H_2O$ [k] NCS (1.0 equiv.) instead of TCCA; [l] NCS (0.4 equiv.) instead of TCCA)

Fig. 2: Table 2 - Optimization of reaction conditions for the side-chain chlorination of toluene ([a] Conversion of the starting material to **4a;** [b] Only here **5** is obtained after 20 h in the ratio 10 : 1 (**4a** : **5**) and disappears over the course of the reaction; [c] without NHPI; [d] NHPI (0.05 equiv.); [e] NHPI (0.2 equiv.); [f] TCCA (0.6 equiv.); [g] TCCA (0.8 equiv.); [h] $H_2O$ (0.1 equiv.) added; [i] $H_2O$ (0.3 equiv.) added; [j] 18°C; [k] 25°C; [l] $Cu(OAc)_2•H_2O$. [m] $Cu(acac)_2$. [n] $CuCl_2$.)

Fig. 3: Samples of Substrate scope for the side-chain chlorination of various toluene derivatives, yields of isolated pure products given. ([a] According to general method P1; [b] Determined by $^{1}H$ NMR; [c] Two equiv. of substrate used)

Fig. 4: Samples of Substrate scope for the chlorination of hydrocarbons; yields of isolated pure products given. According to general method P2. ([a] According to general method P1, without $CBr_4$ ; [b] Determined by $^{1}H$-

NMR)

Fig. 5: Suggested mechanism for the side-chain chlorination of arenes, e.g. toluene

Fig. 6A: Table 3 - Radical chain length initiated by NHPI. ([a] Yield determined by $^1$H-NMR with dimethylterephthalate as internal standard.)

Fig. 6B: Table 4 - Radical chain length initiated by NHPI and $CBr_4$. ([a] Yield determined by $^1$H-NMR with dimethylterephthalate as internal standard.)

Fig. 7: Table 5 - Evaluation of reaction conditions for the side-chain iodination of toluene. ([a] Yield determined by $^1$H-NMR with dimethyl terephthalate (DMT) as internal standard; [b] *N*-iodosuccinimide (0.625 mmol, 1 equiv.) used instead of DIH; [c] 1 mL $CH_2Cl_2$; [d] 1.5 mL $CH_2Cl_2$; [e] 0.08 equiv. NHPI; [f] Average $^1$H-NMR yield out of two runs; [g] 0.25 equiv. NHPI; [h] 0.33 equiv. NHPI)

Fig. 8: Substrate scope for the side-chain iodination of various toluene derivatives; yields of isolated pure products given. (Applied method: $CH_2Cl_2$ (2 mL), DIH (0.375 mmol), *o*-$NO_2C_6H_4CO_2H$ (0.136 mmol), NHPI (0.068 mmol), substrate (2.50 mmol), 25-27 °C.

Fig.9: Substrate scope for the side-chain iodination of toluene derivatives; yields of isolated product given, applied method according to scheme 5. [a] 2.5 mmol substrate used, [b] without acid.

Fig. 10: Reaction mechanism for the side-chain iodination of toluene

Fig. 11A: NMR studies of the complexation of 3-ITMH and acid in $CD_2Cl_2$ (62 mM)

Fig. 11B: Reaction scheme of the evaluation of the role of acid

Fig. 11C: Data of the evaluation of the role of acid; [a] Yield determined by $^1$H NMR with dimethyl terephthalate (DMT) as internal standard; [b] 3h.

Fig. 11D: DOSY NMR of 3-ITMH and *o*-$NO_2C_6H_4CO_2H$

Fig. 11E: Self-diffusion coefficients for pure o-nitrobenzoic acid and 3-ITMH in $CD_2Cl_2$ and in the complex

Fig. 12A: Reaction scheme of the evaluation of the role of $I_2$

Fig. 12B: Data of the evaluation of the role of $I_2$; [a] Yield determined by $^1$H NMR with dimethyl terephthalate (DMT) as internal standard.

Fig. 13A: NHPI-derivates tested as radical initiators.

Fig. 13B: Experimental results of tested NHPI derivatives; [a] $^1$H NMR yield with dimethylterephthalate (DMT) as internal standard; yield based on toluene.

Fig. 14A: Experimental results of the first optimization of the yield with a design of experiments (DoE) approach; [a] $^1$H NMR yield with DMT as internal standard; yield based on TCCA considering all chlorine atoms active (1.50 mmol $Cl^\bullet$).

Fig. 14B: Experimental results of the second optimization of the yield with a DoE approach; [a] $^1$H NMR yield with DMT as internal standard; the yield based on TCCA considering all chlorine atoms active (1.50 mmol $Cl^\bullet$).

Fig. 15A: Interaction plot substrate vs. temperature for Fig. 14A

Fig. 15B: Interaction plot substrate vs. volume of solvent for Fig. 14A

Fig. 15C: Interaction plot temperature vs. volume of solvent for Fig. 14A

Fig. 16A: Parameters of the DoE-Experiments regarding iodination (Screening analysis)

Fig. 16B: Parameters of the DoE-Experiments regarding iodination (Relative relations)

## Claims

1. Process for the production of halogenated hydrocarbons comprising the usage of at least one halogenating agent which contains at least one halogen-atom per formular unit, **characterized in that** at least one educt is reacted with the at least one halogenating agent together with substituted or unsubstituted *N*-hydroxyphthalimide and a metal-containing salt-like compound or a carboxylic or sulfonic acid, whereat the reaction mixture either contains at least one solvent or does not contain a solvent.

2. Process according to claim 1, **characterized in that** the at least one halogen atom per formular unit of the halogenating agent is chosen from the list containing F, Cl, Br, I.

3. Process according to claim 1 or claim 2, **characterized in that** the at least one halogenating agent is chosen from the list comprising *N*-hlorosuccinimide, substituted *N*-chlorosuccinimide, trichloroisocyanuric acid, 1,3-dichloro-hydantoin, substituted 1,3-dichloro-hydantoin, 1-chloro-hydantoin, substituted 1-chloro-hydantoin, 3-chloro-hydantoin, substituted 3-chloro-hydantoin, *N*-bromosuccinimide, substituted *N*-bromosuccinimide, tribromoisocyanuric acid, 1,3-dibromo-hydantoin, substituted 1,3-dibromo-hydantoin, 1-bromo-hydantoin, substituted 1-bromo-hydantoin, 3-bromo-hydantoin, substituted 3-bromo-hydantoin, *N*-iodosuccinimide, substituted *N*-iodosuccinimide, triiodoi-socyanuric acid, 1,3-diiodo-hydantoin, substituted 1,3-diiodo-hydantoin, 1-iodo-hydantoin, substituted 1-iodo-hydantoin, 3-iodo-hydantoin, substituted 3-iodo-hydantoin, whereat the at least one substituent of the substituted *N*-chlorosuccinimide or substituted *N*-bromosuccinimide or substituted *N*-iodosuccinimide or substituted 1,3-dichloro-hydantoin or substituted 1-chloro-hydantoin or substituted 3-chloro-hydantoin or substituted 1,3-dibromo-hydantoin or substituted 1-bromo-hydantoin or substituted 3-bromo-hydantoin or substituted 1,3-diiodo-hydantoin or substituted 1-iodo-hydantoin or substituted 3-iodo-hydantoin is chosen from the list comprising the substituents methyl, ethyl, propyl, isopropyl, n-butyl, *sec*-butyl, *tert*-butyl, phenyl.

4. Process according to claim 1 or claim 2, **characterized in that** the at least one halogenating agent is chosen from the list comprising 1,3-dichloro-5,5-dimethylhydantoin, 1-chloro-3,5,5-trimethylhydantoin, 1-chloro-3,3,4-trimethylhydantoin, 1-chloro-5,5-dimethylhydantoin, 1-chloro-3,3-dimethylhydantoin, 1,3-dibromo-5,5-dimethylhydantoin, 1-bromo-3,5,5-trimethylhydantoin, 1-bromo-3,3,4-trimethylhydantoin, 1-bromo-5,5-dimethylhydantoin, 1-bromo-3,3-dimethylhydantoin, 1,3-diiodo-5,5-dimethylhydantoin, 1-iodo-3,5,5-trimethylhydantoin, 1-iodo-3,3,4-trimethylhydantoin, 1-iodo-5,5-dimethylhydantoin, 1-iodo-3,3-dimethylhydantoin.

5. Process according to one of the preceding claims or a combination of at least two of the preceding claims, **characterized in that** the metal-containing salt-like compound contains at least one metal-atom chosen from the metals of the groups 6, 7, 8, 9, 10, 11 or 12 of the periodic system of chemical elements.

6. Process according to claim 5, **characterized in that** the metal-containing salt-like compound is either cobalt(II)acetate, $Co(OAc)_2$, or copper(II)acetate, $Cu(OAc)_2$.

7. Process according to one of the preceding claims 1 through 4 or a combination of at least two of the preceding claims 1 through 4, **characterized in that** the carboxylic or sulfonic acid exhibits a $pK_a$ value between -3 and 6, more preferred between -2 and 5, and most preferred between 3.5 and 1.2.

8. Process according to claim 7, **characterized in that** the carboxylic or sulfonic acid is chosen from the list comprising the acids benzoic acid, acetic acid, Diphenic acid, p-nitrobenzoic acid, o-nitrobenzoic acid, Camphor sulfonic acid, trifluoroacetic acid, p-toluenesulfonic acid.

9. Process according to any one of the preceding claims, **characterized in that** the substituted *N*-hydroxyphthalimide contains at least one substituent chosen from the list comprising the substituents methyl, ethyl, propyl, isopropyl, n-butyl, *sec*-butyl, *tert*-butyl, phenyl, F, Cl, Br, I, $CF_3$, $NO_2$, CN, $CO_2Me$, $CO_2H$, OAc, $NHR_d$, NHAc, OH, OMe, whereat $R_d$ is chosen from the List comprising methyl, ethyl, propyl, *i*-propyl, butyl, *sec*-butyl, *tert*-butyl.

10. Process according to any one of the preceding claims, **characterized in that** the amount of substituted or unsubstituted *N*-hydroxyphthalimide lies between 0.1 mol% and 50 mol%, more preferred between 1 mol% and 40 mol%, even more preferred between 2 mol% and 30 mol% and most preferred between 3 mol% and 20 mol% in relation to the educt or the halogenating or pseudo-halogenating agent.

**11.** Process according to any one of the preceding claims, **characterized in that** the reaction mixture also contains a certain amount of $CBr_4$ between 0.1 mol% and 50 mol%, more preferred between 1 mol% and 30 mol%, even more preferred between 5 mol% and 20 mol% and most preferred between 7 mol% and 15 mol% in relation to the educt or the halogenating agent.

**12.** Process according to any one of the preceding claims, **characterized in that** the concentration of the metal containing salt-like compound or the carboxylic or sulfonic acid in the reaction mixture is between 0.2 mmol/mL and 200 mmol/mL, more preferred between 0.3 mmol/mL and 25 mmol/mL.

**13.** Usage of the process according to any one of the preceding claims for the production of alkyl halides or benzyl halides or allyl halides.

Fig. 1

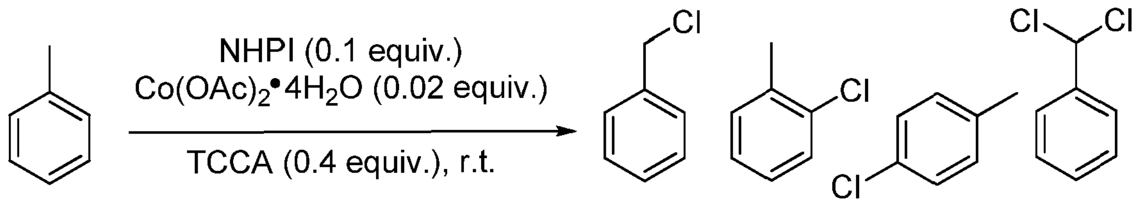


**3** **4a** **4b** **4c** **4d**

| Entry | *t* [h] | Solvent | Conv. [%][a] | 4a[b] | 4b[b] | 4c[b] | 4d[b] |
|---|---|---|---|---|---|---|---|
| 1[c,d] | 16 | $CH_2Cl_2$ | 33 | 73 | 12 | 15 | – |
| | 19 | | 81 | 41 | 25 | 34 | – |
| 2[c] | 16 | AcOH | 78 | 30 | 51 | 19 | – |
| 3[e] | 18 | $CH_2Cl_2$ | 28 | 86 | 7 | 7 | – |
| 4 | 5 | MeCN | 80 | 16 | 36 | 48 | – |
| 5[f] | 16 | $CHCl_3$ | 48 | 98 | 2 | traces | – |
| | 40 | | 46 | 98 | 2 | traces | – |
| 6 | 16 | 1,2-DCE | 46 | 100 | – | – | – |
| | 40 | | 35 | 100 | – | – | – |
| 7 | 16 | $CCl_4$ | 45 | 100 | – | – | – |
| | 40 | | 93 | 82 | – | – | 18 |
| 8[g] | 24 | $CCl_4$ | 52 | 96 | – | – | 4 |
| | 48 | | 91 | 88 | – | – | 12 |
| 9[h] | 24 | $CCl_4$ | 81 | 90 | – | – | 10 |
| | 48 | | 89 | 82 | – | – | 18 |
| 10[i] | 24 | $CCl_4$ | 11 | 100 | – | – | – |
| | 48 | | 51 | 100 | – | – | – |
| 11[j] | 46 | $CCl_4$ | – | – | – | – | – |
| 12[k] | 48 | $CCl_4$ | – | – | – | – | – |
| 13[l] | 48 | $CCl_4$ | – | – | – | – | – |

Fig. 2

| Entry | *t* [h] | Additive [equiv.] | Conv. [%][a] |
|---|---|---|---|
| 1 | 20 | CBr4 (1.0) | 53[b] |
| | 43 | | 65 |
| 2 | 17 | – | 31 |
| | 42 | | 64 |
| 3 | 16 | $CBr_4$ (0.01) | 33 |
| | 40 | | 58 |
| 4 | 16 | $CBr_4$ (0.05) | 39 |
| | 40 | | 63 |
| 5 | 16 | $CBr_4$ (0.1) | 69 |
| | 40 | | 79 |
| 6 | 16 | $CBr_4$ (0.15) | 47 |
| | 40 | | 58 |
| 7 | 16 | $CBr_4$ (0.2) | 24 |
| | 14 | | 49 |
| 8 | 16 | $HCBr_3$ (0.1) | 25 |
| | | | 45 |
| 9 | 16 | $HCBr_3$ (0.15) | 28 |
| | 40 | | 54 |
| 10 | 16 | $HCBr_3$ (0.2) | 30 |
| | 40 | | 55 |
| 11 | 16 | $HCBr_3$ (0.5) | 47 |
| | 40 | | 63 |
| 12 | 16 | $HCCl_3$ (0.1) | 22 |
| | | | 45 |
| 13[c] | 40 | $CBr_4$ (0.1) | – |
| 14[d] | 40 | $CBr_4$ (0.1) | – |
| 15[e] | 40 | $CBr_4$ (0.1) | 77 |
| 16[f] | 41 | $CBr_4$ (0.1) | 33 |
| 17[g] | 41 | $CBr_4$ (0.1) | 24 |
| 18[h] | 16 | $CBr_4$ (0.1) | 16 |
| | 40 | | 44 |
| 19[i] | 16 | $CBr_4$ (0.1) | 1 |
| | 40 | | 37 |
| 20[j] | 18 | $CBr_4$ (0.1) | 44 |
| 21[k] | 19 | $CBr_4$ (0.1) | 74 |
| 22[l] | 16 | $CBr_4$ (0.1) | 47 |
| | 40 | | 75 |
| 23[m] | 16 | $CBr_4$ (0.1) | 50 |
| | 40 | | 62 |
| 24[n] | 16 | $CBr_4$ (0.1) | 34 |
| | 40 | | 79 |

Fig. 3

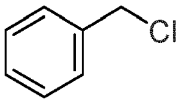

**4a**
**58%** (22 h)

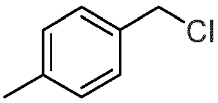

**6a**
**31%** (22 h)

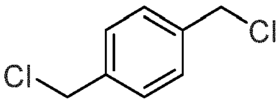

**6b**
**12%**

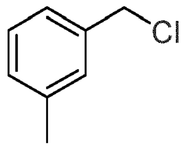

**7**
**33%** (42 h)

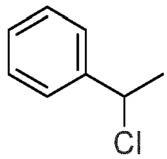

**8**
**26%/36%**[a] (22 h)

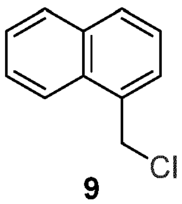

**9**
**15%** (21 h)

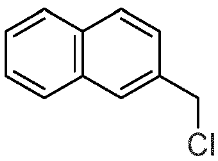

**10**
**7%**[b] (21 h)

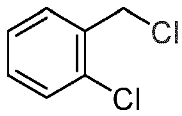

**11**
**67%** (40 h)

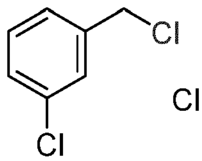

**12**
**55%** (40 h)

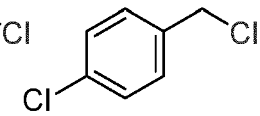

**13**
**73%** (17 h)

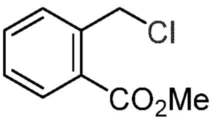

**14**
**61%** (24 h)

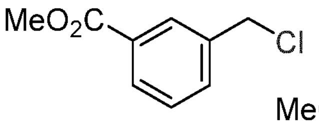

**15**
**50%** (19 h)

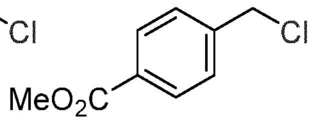

**16**
**85%** (24 h)

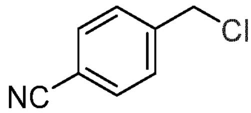

**17**
**59%/70%**[c] (24 h)

Fig. 4

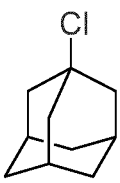

4 h
**18a, 13%**[a,b]

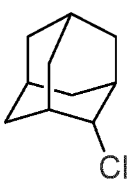

**18b, 10%**

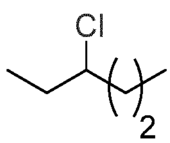

40 °C, 96 h
**19a, 13%**[b]

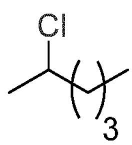

**19b, 13%**

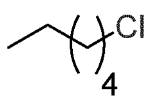

**19c, 4%**

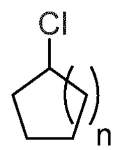

30 °C, 20–64 h
n = 1: **20, 31%**
n = 2: **21, 38%**
n = 4: **22, 24%**

Fig. 5

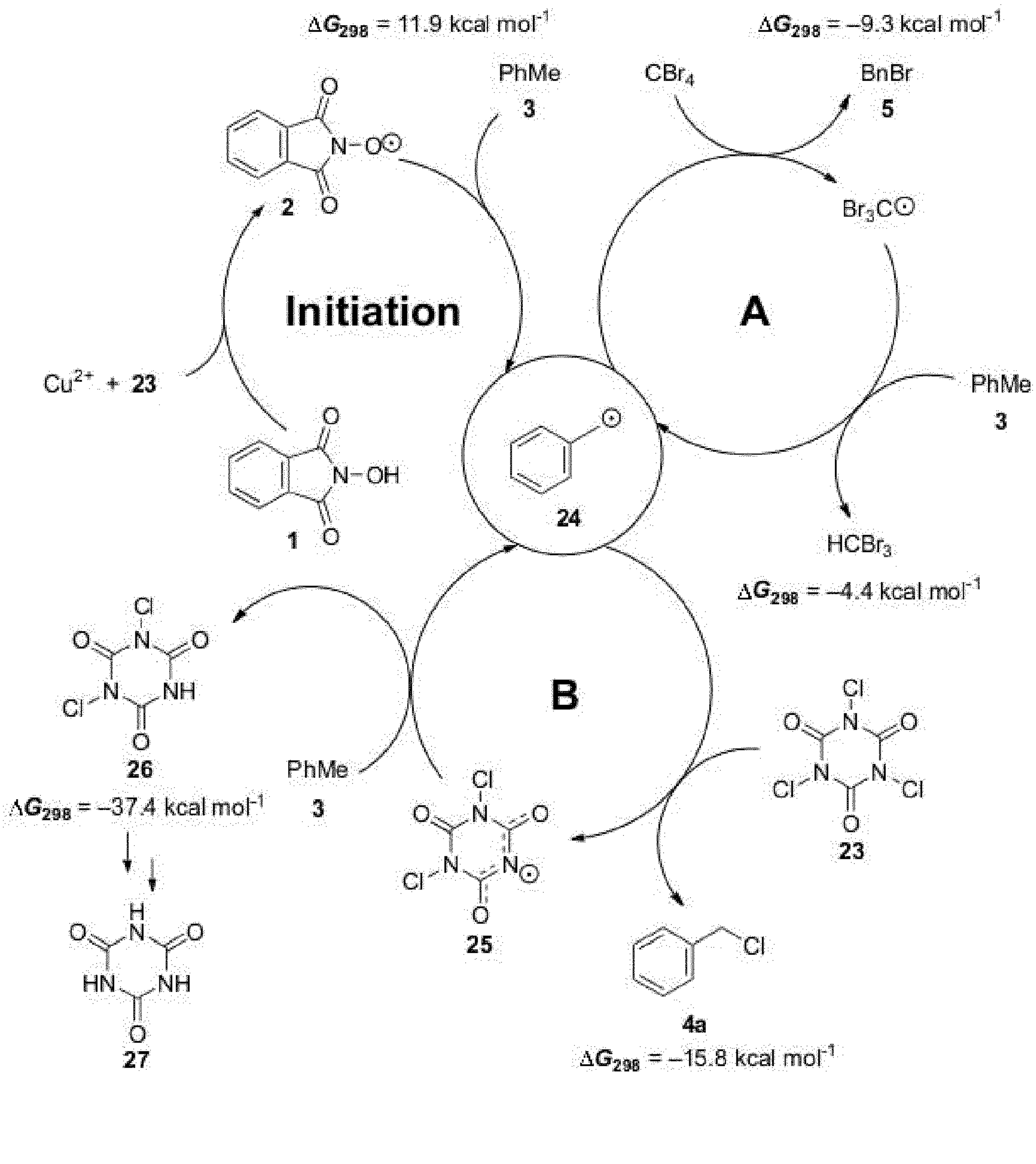
ΔG298 = 11.9 kcal mol-1
PhMe
3
2
Initiation
Cu2+ + 23
1
24
ΔG298 = –9.3 kcal mol-1
CBr4
BnBr
5
Br3C⊙
A
PhMe
3
HCBr3
ΔG298 = –4.4 kcal mol-1
B
26
ΔG298 = –37.4 kcal mol-1
27
PhMe
3
25
23
4a
ΔG298 = –15.8 kcal mol-1

Fig. 6A

| 1 h | | | |
|---|---|---|---|
| NHPI [equiv.] | **13** [%][a] | Chain length | Average chain length |
| 0.1 | 27 | 3 | 1.2 (1.8) |
| 0.2 | 31 | 1.5 | |
| 0.3 | 31 | 1 | |
| 0.4 | 32 | 0.8 | |
| 0.5 | 31 | 0.6 | |
| 0.6 | 31 | 0.5 | |
| | | | |
| 3 h | | | |
| 0.1 | 35 | 3.5 | 1.5 (2.2) |
| 0.2 | 37 | 1.9 | |
| 0.3 | 38 | 1.3 | |
| 0.4 | 37 | 0.9 | |
| 0.5 | 42 | 0.8 | |
| 0.6 | 33 | 0.5 | |

Fig. 6B

| 1 h | | | | |
|---|---|---|---|---|
| NHPI [equiv.] | $CBr_4$ [equiv.] | 13 [%][a] | Chain length | Average chain length |
| 0.1 | 0.1 | 32 | 3.2 | (1.9) |
| 0.2 | 0.2 | 29 | 1.45 | |
| 0.3 | 0.3 | 32 | 1.06 | |
| | | | | |
| 3 h | | | | |
| 0.1 | 0.1 | 42 | 4.2 | (2.6) |
| 0.2 | 0.2 | 40 | 2 | |
| 0.3 | 0.3 | 43 | 1.4 | |

Fig. 7

| Entry | t [h] | Substrate [equiv.] | Additive [equiv.] | BnI [%][a] | BnCHO [%][a] |
|---|---|---|---|---|---|
| 1[b] | 16 | 1.0 | $Cu(OAc)_2$*$H_2O$ (0.03) | 11 | 3 |
| 2 | 16 | 1.7 | $Cu(OAc)_2$*$H_2O$ (0.03) | 19 | 3 |
| 3[c] | 16 | 1.7 | – | 18 | 2 |
| 4[d] | 16 | 1.7 | – | 27 | 3 |
| 5[e] | 16 | 1.7 | – | 13 | <1 |
| 6[f] | 16 | 1.7 | – | 33 | 3 |
| 7[g] | 16 | 1.7 | – | 38 | 1 |
| 8[h] | 16 | 1.7 | – | 39 | 1 |
| 9 | 16 | 3.4 | – | 60 | 3 |
| 10 | 16 | 6.8 | – | 69 | 1 |
| 11 | 2.5 | 6.8 | – | 8 | – |
| 12 | 2.5 | 6.8 | $BnCO_2H$ (0.18) | 36 | traces |
| 13 | 2.5 | 6.8 | $BnCO_2H$ (0.36) | 63 | 2 |
| 14 | 2.5 | 6.8 | 100% AcOH (0.36) | 62 | 1 |
| 15 | 2.5 | 6.8 | $Ac_2O$ | 13 | 1 |
| 16 | 2.5 | 6.8 | Diphenic acid (0.36) | 65 | 1 |
| 17 | 2.5 | 6.8 | *p*-$NO_2C_6H_4CO_2H$ (0.36) | 15 | <1 |
| 18 | 2.5 | 6.8 | *o*-$NO_2C_6H_4CO_2H$ (0.36) | 69 | <1 |
| 19 | 2.5 | 6.8 | $Cl_2HCO_2H$ (0.36) | 47 | <1 |
| 20 | 2.5 | 6.8 | Camphor sulfonic acid (0.36) | 12 | 1 |
| 21 | 2.5 | 6.8 | *p*-TsOH (0.36) | 12 | 1 |
| 22 | 2.5 | 6.8 | TFA (0.36) | 21 | <1 |
| 23 | 2.5 | 6.8 | MsOH (0.36) | – | <1 |
| 24 | 2.5 | 6.8 | $Ph_3PS$ (0.36) | – | – |

Fig. 8

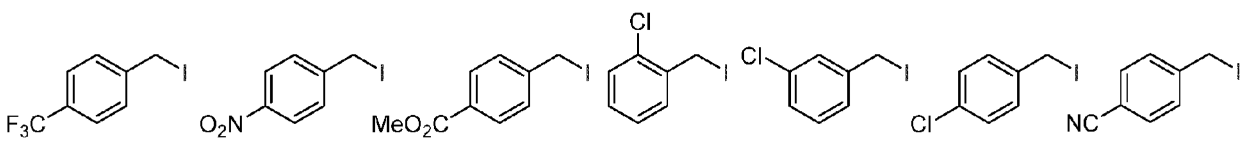

**28** **48%** (19 h) **29** **61%** (22 h) **30** **65%** **31** **67%** **32** **49%** (23 h) **33** **25%** **34** **35%**

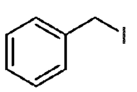

**35**
**60%**

Fig. 9

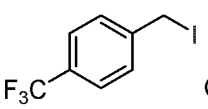
28
**A** 37% (19 h)
**B** 68%

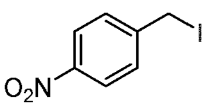
29
**A** 61% (22 h)
**B** 72%[a]

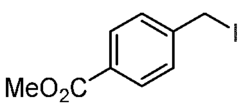
30
**A** 65%
**B** 65%[a]

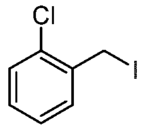
31
**A** 67%
**B** 64%

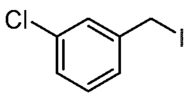
32
**A** 49% (23 h)
**B** 59%

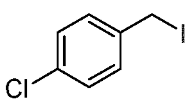
33
**A** 49%
**B** 64%

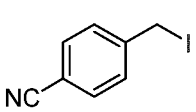
34
**A** 35%
**B** 50%

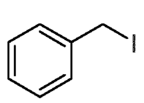
35
**A** 60%
**B** 67%

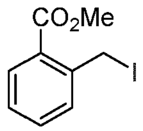
36
**A** 48%
**B** 55%[a]

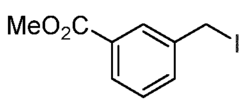
37
**A** 51%
**B** 55%[a]

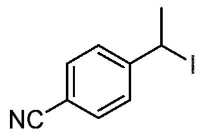
38
**A** 69% (17 h)
**B** 59% (17 h)[a,b]

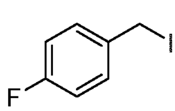
39
**A** 40%
**B** 68%

Fig. 10

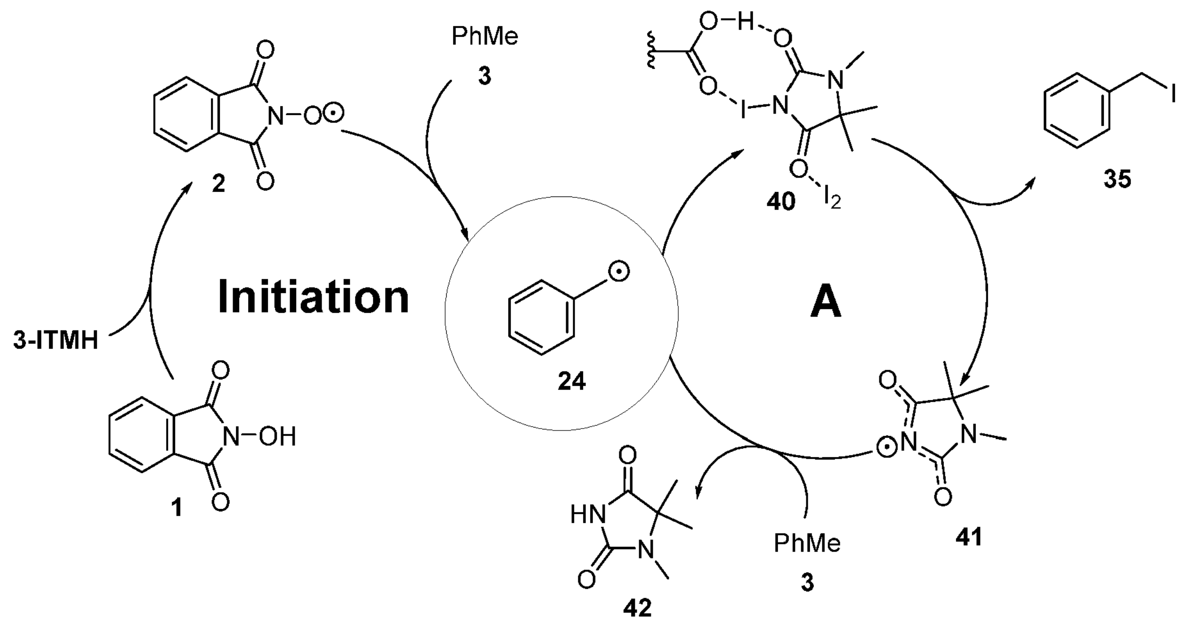

Fig. 11A

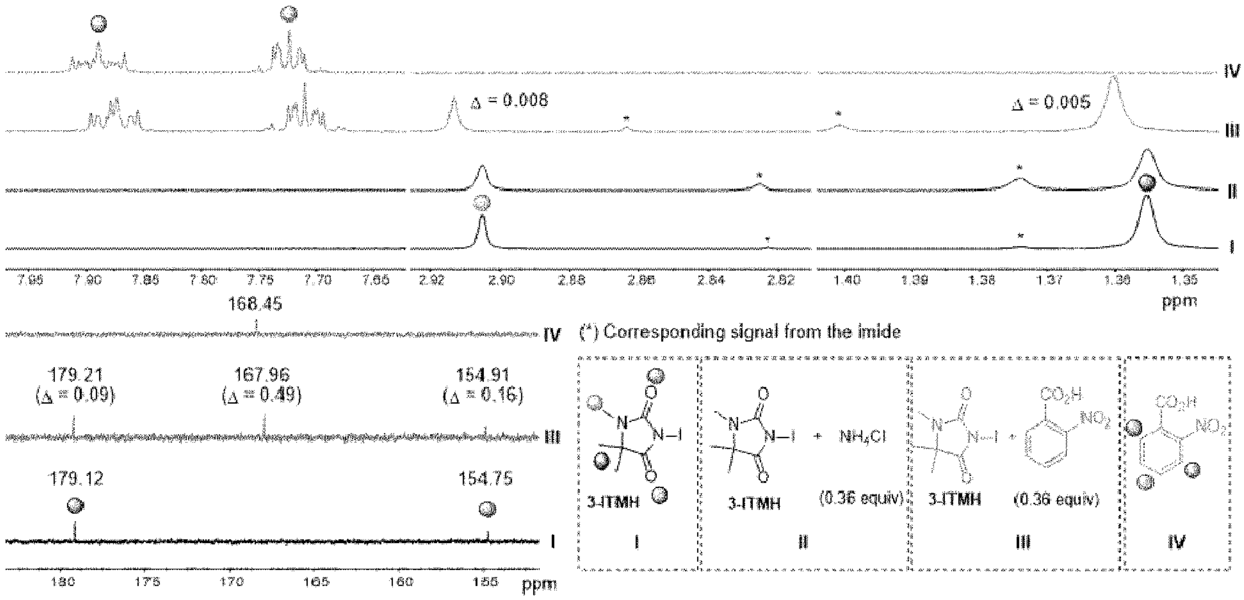

Δ = 0.008
Δ = 0.005
IV
III
II
I
ppm
168.45
179.21 (Δ = 0.09)
167.96 (Δ = 0.49)
154.91 (Δ = 0.16)
179.12
154.75
(*) Corresponding signal from the imide
3-ITMH
NH4Cl
(0.36 equiv)

Fig. 11B

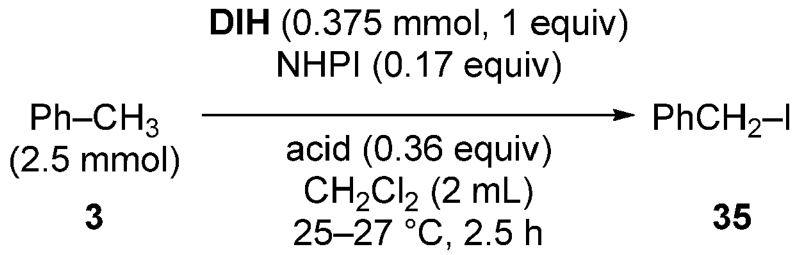


Fig. 11C

| no. | Acid | p$K_a$ (DMSO) | **35** (%)[a] |
|---|---|---|---|
| 1 | – | – | 8 |
| 2 | *o*-$NO_2C_6H_4CO_2H$ | 9.0 | 69 |
| 3 | $NH_4Cl$ | 10.5 | 56[b] |
| 4 | $PhCO_2H$ | 11.1 | 63 |

Fig. 11D

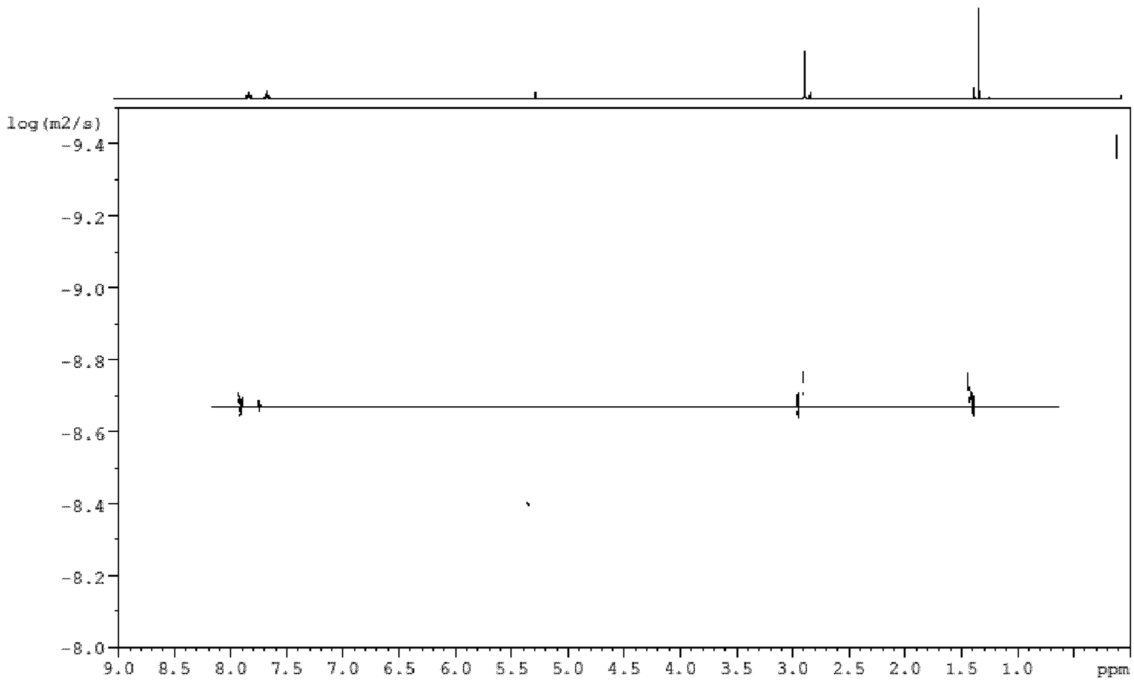

Fig. 11E

| Molecule | D(m²/s) | |
|---|---|---|
| | Pure in $CD_2Cl_2$ | In the complex in $CD_2Cl_2$ |
| *o*-nitrobenzoic acid | $3{,}98 \cdot 10^{-9}$ | $1{,}99 \cdot 10^{-9}$ |
| 3-ITMH | $3.16 \cdot 10^{-9}$ | $1.99 \cdot 10^{-9}$ |

Fig. 12A

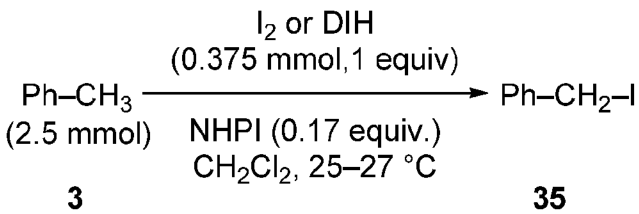


**3** **35**

Fig. 12B

| no. | t [h] | Iodine source | Additive (equiv) | **35** (%)a |
|---|---|---|---|---|
| 1 | 15 | $I_2$ | – | – |
| 2 | 24 | $I_2$ | *o*-$NO_2C_6H_4CO_2H$ (0.36) | – |
| 3 | 3 | DIH | – | 8 |
| 4 | 3 | DIH | $I_2$ (0.1) | 13 |

Fig. 13A

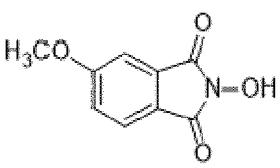
**40**

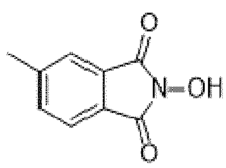
**41**

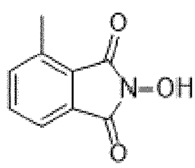
**42**

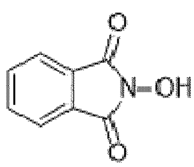
**43**

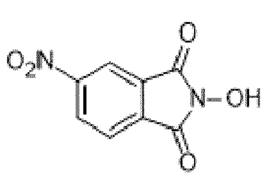
**44**

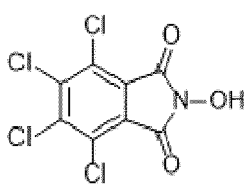
**45**

Fig. 13B

| NHPI-derivative | Benzyl chloride [%][a] | Benzal chloride [%][a] |
|---|---|---|
| **40** | 60 | 7 |
| **41** | 38 | 2 |
| **42** | 44 | 4 |
| **43** | 59 | 8 |
| **44** | 33 | 3 |
| **45** | 65 | 7 |

Fig. 14A

| # | PhMe [mmol] | T [°C] | V($CH_2Cl_2$) [mL] | Benzyl chloride [%][a] | Benzal chloride [%][a] |
|---|---|---|---|---|---|
| 1 | 1.50 | 25 | 2 | 42 | 5 |
| 2 | 1.50 | 40 | 2 | 47 | 5 |
| 3 | 15.0 | 25 | 2 | 65 | <1 |
| 4 | 15.0 | 40 | 2 | 75 | <1 |
| 5 | 15.0 | 25 | 6 | 61 | <1 |
| 6 | 15.0 | 40 | 6 | 56 | <1 |
| 7 | 1.50 | 25 | 6 | 46 | 4 |
| 8 | 1.50 | 40 | 6 | 58 | 4 |
| 9 | 7.50 | 33 | 4 | 65 | 1 |

Fig. 14B

| # | PhMe [mmol] | V($CH_2Cl_2$) [mL] | Benzyl chloride [%][a] | Benzal chloride [%][a] |
|---|---|---|---|---|
| 1 | 5 | 0.5 | 73 | 1 |
| 2 | 15 | 0.5 | 73 | <1 |
| 3 | 5 | 1.5 | 71 | 1 |
| 4 | 15 | 1.5 | 70 | <1 |
| 5 | 10 | 0.75 | 71 | <1 |

Fig. 15A

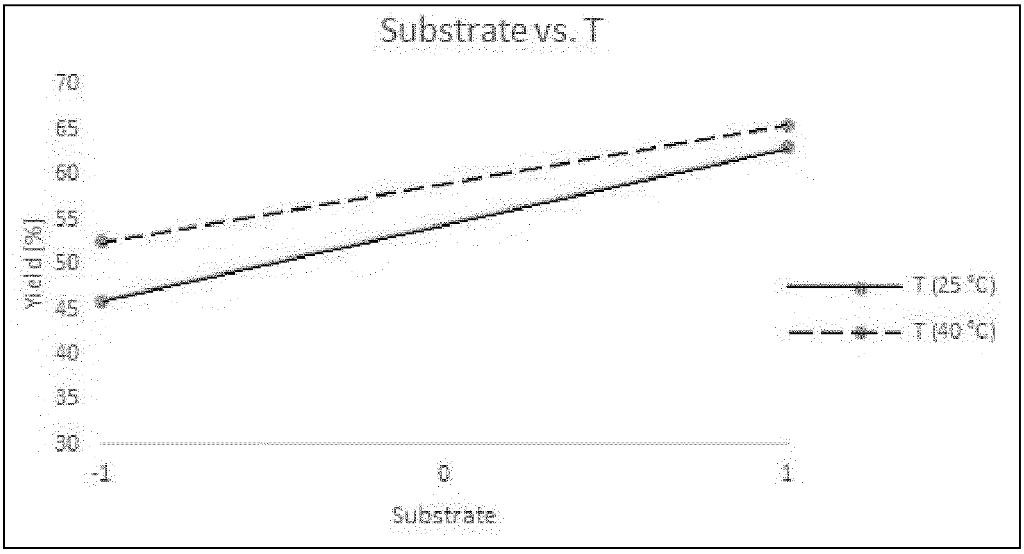


Fig. 15B

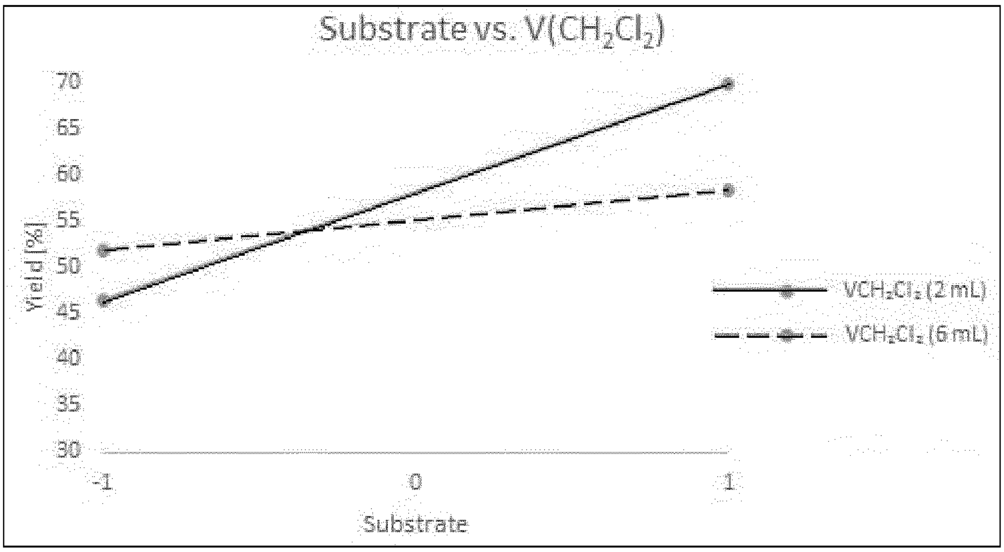

Fig. 15C

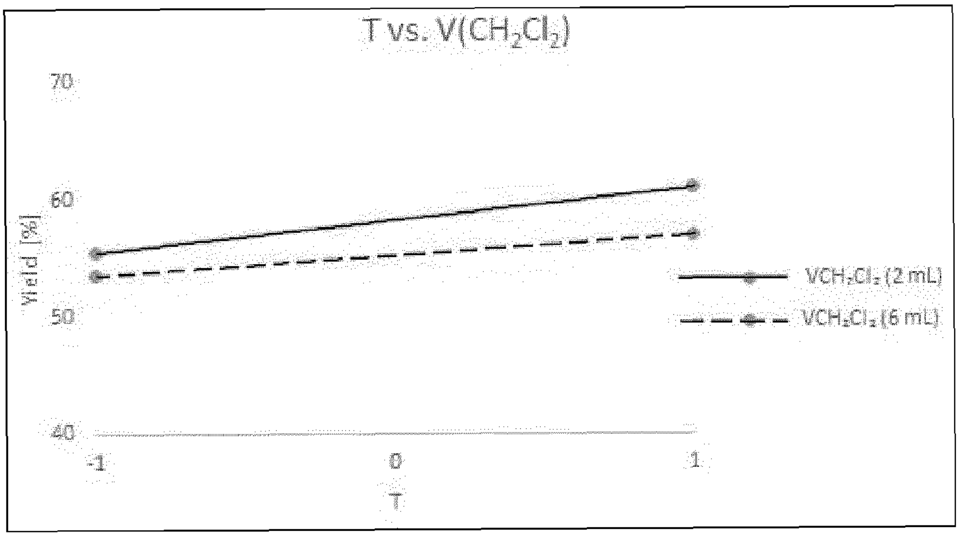


Fig. 16A

| Dilution (mL) | NHPI (equiv) | Substrate (equiv) | Yield (%) |
|---|---|---|---|
| 1 | 0.17 | 1.7 | 18 |
| 1.5 | 0.17 | 1.7 | 27 |
| 2 | 0.17 | 1.7 | 33 |
| 2 | 0.08 | 1.7 | 13 |
| 2 | 0.25 | 1.7 | 38 |
| 2 | 0.34 | 1.7 | 39 |
| 2 | 0.17 | 3.4 | 60 |
| 2 | 0.17 | 6.8 | 69 |

| Substrate (equiv) | Yield (%) | Slope |
|---|---|---|
| 1.7 | 28 | 16 |
| 3.4 | 60 | |
| 6.8 | 69 | 4.5 |

| NHPI (equiv) | Yield (%) | Slope |
|---|---|---|
| 0.08 | 13 | 10 |
| 0.17 | 33 | |
| 0.25 | 38 | 2.5 |

| Dilution (mL) | Yield (%) | Slope |
|---|---|---|
| 1 | 18 | 4.5 |
| 1.5 | 27 | |
| 2 | 33 | 3 |

| Dilution/NHPI | Yield (%) | Slope |
|---|---|---|
| 2/0.17 | 54 | 7.5 |
| 2/0.34 | 39 | |

| Dilution/Substrate | Yield (%) | Slope |
|---|---|---|
| 2/1.7 | 31 | 14.6 |
| 2/3.4 | 60 | |

| NHPI/Substrate | Yield (%) | Slope |
|---|---|---|
| 0.17/1.7 | 26 | 6 |
| 0.25/1.7 | 38 | |

Fig. 16B

| Dilution/NHPI | Dilution/Substrate | NHPI/Substrate | Substrate | NHPI | Dilution |
|---|---|---|---|---|---|
| 1.3 | 2.4 | 1.0 | 1.8 | 1.0 | 1.2 |

Europäisches Patentamt
European Patent Office
Office européen des brevets

# EUROPEAN SEARCH REPORT

Application Number
EP 17 18 6879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | MINISCI F ET AL: "New free-radical halogenations of alkanes, catalysed by N-hydroxyphthalimide. Polar and enthalpic effects on the chemo- and regioselectivity", TETRAHEDRON LETTERS, ELSEVIER, AMSTERDAM, NL, vol. 45, no. 8, 16 February 2004 (2004-02-16), pages 1607-1609, XP004487110, ISSN: 0040-4039, DOI: 10.1016/J.TETLET.2003.12.130 * schemes (1), (2); page 1609; table 1 * ----- | 1-13 | INV. C07C17/00 C07C17/14 C07C19/01 C07C19/07 C07C22/02 C07C22/04 C07C23/08 C07C23/10 C07C23/16 C07C23/28 C07C255/50 C07C69/78 C07C205/06 C07C67/307 C07C201/12 C07B39/00 C07C253/30 |
| Y | ULF TILSTAM ET AL: "TRICHLOROISOCYANURIC ACID: A SAFE AND EFFICIENT OXIDANT", ORGANIC PROCESS RESEARCH AND DEVELOPMENT, CAMBRIDGE, GB, vol. 6, no. 4, 1 January 2002 (2002-01-01), pages 384-393, XP002334135, DOI: 10.1021/OP010103H * schemes 6-18 * ----- | 1-8, 10-13 | TECHNICAL FIELDS SEARCHED (IPC) C07C C07B |
| Y | GÜNTER E. JEROMIN ET AL: "Seitenkettenchlorierungen von N-Heterocyclen mit Trichlorisocyanursäure (TCC)", CHEMISCHE BERICHTE, vol. 120, no. 4, 1 April 1987 (1987-04-01), pages 649-651, XP055335522, DE ISSN: 0009-2940, DOI: 10.1002/cber.19871200431 * scheme 1 * ----- -/-- | 1-8, 10-13 | |

1 The present search report has been drawn up for all claims

| Place of search | Date of completion of the search | Examiner |
|---|---|---|
| Munich | 19 October 2017 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 17 18 6879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2015/068159 A2 (TECHNION RES & DEV FOUNDATION [IL]) 14 May 2015 (2015-05-14)<br>* examples 18, 19 *<br>----- | 1-8, 10-13 | |
| Y | KIYOSHI TANEMURA ET AL: "Halogenation of Aromatic Compounds by N-chloro-, N-bromo-, and N-iodosuccinimide",<br>CHEMISTRY LETTERS, CHEMICAL SOCIETY OF JAPAN, JAPAN,<br>vol. 32, no. 10,<br>8 September 2003 (2003-09-08), pages 932-933, XP002530033,<br>ISSN: 0366-7022, DOI: 10.1246/CL.2003.932<br>* page 933, left-hand column *<br>----- | 1-8, 10-13 | |
| A | CN 101 318 903 A (YANTAI INSITUTE OF COASTAL ZON [CN])<br>10 December 2008 (2008-12-10)<br>* embodiments 6-7 *<br>----- | 1-13 | |
| Y | WENTZEL BASTIENNE B ET AL: "N-Hydroxyphthalimide/Cobalt(II) Catalyzed Low Temperature Benzylic Oxidation Using Molecular Oxygen",<br>TETRAHEDRON, ELSEVIER SCIENCE PUBLISHERS, AMSTERDAM, NL,<br>vol. 56, no. 39,<br>22 September 2000 (2000-09-22), pages 7797-7803, XP085020315,<br>ISSN: 0040-4020, DOI: 10.1016/S0040-4020(00)00679-7<br>* compounds 1a, 1b, 1c, 1f, 1h *<br>-----<br>-/-- | 9 | TECHNICAL FIELDS SEARCHED (IPC) |

The present search report has been drawn up for all claims

1

| Place of search | Date of completion of the search | Examiner |
|---|---|---|
| Munich | 19 October 2017 | Sáez Díaz, R |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

# EUROPEAN SEARCH REPORT

Application Number
EP 17 18 6879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KAZUHIRO SUGAMOTO ET AL: "Microwave-assisted Synthesis of N -Hydroxyphthalimide Derivatives", SYNTHETIC COMMUNICATIONS, vol. 35, no. 1, 1 January 2005 (2005-01-01), pages 67-70, XP055414738, PHILADELPHIA, PA; US ISSN: 0039-7911, DOI: 10.1081/SCC-200046498 * compound 2h * ----- | 9 | |
| | | | TECHNICAL FIELDS SEARCHED (IPC) |

The present search report has been drawn up for all claims

1

| Place of search | Date of completion of the search | Examiner |
|---|---|---|
| Munich | 19 October 2017 | Sáez Díaz, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 17 18 6879

This annex lists the patent family members relating to the patent documents cited in the above-mentioned European search report.
The members are as contained in the European Patent Office EDP file on
The European Patent Office is in no way liable for these particulars which are merely given for the purpose of information.

19-10-2017

| Patent document cited in search report | Publication date | Patent family member(s) | Publication date |
|---|---|---|---|
| WO 2015068159 A2 | 14-05-2015 | EP 3066081 A2 | 14-09-2016 |
| | | IL 229326 A | 30-11-2015 |
| | | JP 2016536309 A | 24-11-2016 |
| | | US 2016272598 A1 | 22-09-2016 |
| | | WO 2015068159 A2 | 14-05-2015 |
| CN 101318903 A | 10-12-2008 | NONE | |

EPO FORM P0459

For more details about this annex : see Official Journal of the European Patent Office, No. 12/82